# EUROPEAN PATENT SPECIFICATION

(11) **EP 2 120 950 B1**
(45) Date of publication and mention of the grant of the patent: **04.07.2012**
(21) Application number: 08718011.3
(22) Date of filing: 19.03.2008
(51) Int. Cl.: A61K 31/496, A61P 29/00, A61K 31/4709

(54) **USE OF QUINOLINE DERIVATIVES IN THE TREATMENT OF PAIN**
VERWENDUNG VON CHINOLIN-DERIVATEN ZUR BEHANDLUNG VON SCHMERZEN
UTILISATION DE DERIVES QUINOLEINES POUR LE TRAITEMENT DE LA DOULEUR

(30) Priority: 21.03.2007 GB 0705424; 26.06.2007 GB 0712390
(43) Date of publication of application: 25.11.2009
(73) Proprietor: Glaxo Group Limited, Greenford, Middlesex UB6 0NN (GB)
(72) Inventor: BRUTON, Gordon, Hertfordshire SG1 2NY (GB); ORLEK, Barry, Sidney, Harlow Essex CM19 5AW (GB); STEMP, Geoffrey, Hertfordshire SG1 2NY (GB)
(74) Representative: Knight, Lucie Viktoria
(86) International application number: PCT/EP2008/053285
(87) International publication number: WO 2008/113818

(56) References cited:
- WO-A-03/080580
- WO-A-2005/121140
- WO-A-2006/053785
- "The Merck Manual of Diagnosis and Therapy" 2006, MERCK RESEARCH LABORATORIES , XP002487531 page 1769 - page 1781

## Description

### Background of the Invention

Effective pain treatment is an ongoing concern for medicine requiring many different pharmaceutical approaches to suit the cause of the pain and the individual patient.

Pain itself is a broad term and includes many different types of pain, for example: acute pain caused by tissue damage, infection and/or inflammation; chronic pain; somatic pain originating from ligaments, tendons, bones, blood vessels or nerves; visceral pain originating from the body's organs and internal cavities; phantom limb pain; and neuropathic pain which can occur as a result of injury or disease to the nerve tissue itself.

Any pharmaceutical compound effective in treating one or more of these pain types is therefore of great value in controlling the physical and psychological effects of these diseases.

Visceral pain is one of the most common forms of pain produced by disease and one of the most frequent reasons why patients seek medical attention. Visceral pain is commonly associated with Irritable Bowel Syndrome (IBS), the most frequent features of which are recurrent abdominal pain and discomfort, altered bowel habits and a strong female predominance. The definition and criteria for IBS have been formalised by the Rome III criteria, Drossman et al., J. Gastrointestin Liver Dis. 2006; 15(3): 237-241.

A number of pharmaceutical compounds are known to have some effect in the treatment of IBS, Tack et al., Aliment. Pharmacol. Ther. 2006; 24: 183-205. For example, alosetron is a 5-HT₃ antagonist known to be effective in the treatment of abdominal pain and discomfort and bowel-related symptoms in female IBS patients, Camilleri et al., Aliment. Pharmacol. Ther. 1999; 13: 1149-1159.

In addition, amitriptyline is a tricyclic antidepressant drug which has also been used as an IBS treatment. A study by Poitras et al., Digestive Diseases and Sciences 2002; 47(4): 914-920, suggested that amitriptyline was effective in decreasing the clinical symptomatology of I BS and that this clinical improvement was correlated to the modulation of visceral pain perception.

Gabapentin is widely used as a medication to relieve pain. In addition, gabapentin has been shown to reduce rectal sensory thresholds through attenuating rectal sensitivity to distension and enhancing rectal compliance in diarrhoea-predominant IBS patients, Lee et al., Aliment. Pharmacol. Ther. 2005; 22: 981-988.

Inflammation is a common cause of acute pain. Celecoxib is a non-steroidal antiinflammatory drug (NSAID) which binds selectively to the COX-2 isoform of cyclooxygenase in order to reduce inflammation and thereby treat acute inflammatory pain. Clinical studies have demonstrated that celecoxib is effective in the treatment of acute pain and inflammatory pain associated with osteoarthritis and rheumatoid arthritis, Clemett and Goa, Drugs 2000; 59(4): 957-980.

Animal models of human disease are commonly used to predict the effectiveness of a test pharmaceutical compound in treating humans. One such animal model is the intra rectal mustard oil model of visceral pain described by Laird et al. in Pain 2001; 92: 335-342. Mustard oil has been used in a variety of conscious and anaesthetised models to elicit pain or stimulate nociceptive pathways.

A useful animal model for inflammatory pain is the Freund's Complete Adjuvant (FCA)-induced inflammation model. A similar model using carrageenan rather than FCA is described by Clayton et al. in Br. J. Pharmacol. 1997; 120, 219P. By measuring hypersensitivity 24 hours post intra-plantar FCA injection, the effectiveness of a potential analgesic in reversing FCA-induced hypersensitivity can be assessed in a short term model of inflammatory pain. Alternatively, by measuring hypersensitivity post intra-articular injection of FCA into the left knee over a longer time course, for example 13 to 17 days post injection, the effectiveness of a potential analgesic in reversing FCA-induced hypersensitivity can be assessed in a joint pain model of chronic inflammatory pain. Intra-articular FCA injection into the knee rather than intra-plantar injection makes the joint pain model of chronic inflammatory pain more physiologically relevant to chronic human inflammatory disease, for example arthritis, a painful condition in humans that often affects the knee joint.

A useful model for neuropathic pain is the Chronic Constriction Injury (CCI) model of nerve damage-induced neuropathic pain in rats (Kajander, KC et al. (1990) Peptides, 11, 719-728; Wakisaka, S et al. (1992) Brain Research 598 (1-2), 349-352; Mao, J et al. (1993) J. Neurophysiol., 70, 470-481).

It is thus an object of the present invention to find alternative pharmaceutical compounds for use in the treatment of pain and/or IBS.

### Summary of Invention

In a first aspect of the invention, there is therefore provided a method of treatment of pain in mammals, which method comprises the administration to the mammal in need of such treatment, an effective amount of a compound of formula (I): 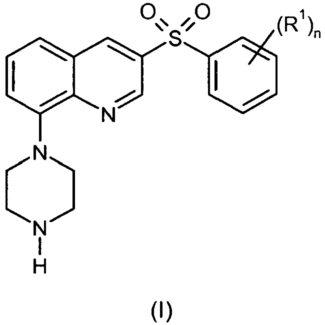 or a pharmaceutically acceptable salt thereof, wherein:
R¹ represents a halogen atom; and
n represents 0, 1, 2 or 3.

In one embodiment of the invention, R¹ represents a fluorine atom.

In a further embodiment of the invention, n represents 0 or 1.

In one particular embodiment of the invention, the compound of formula (I) is 3-phenylsulfonyl-8-piperazin-1-yl-quinoline, or a pharmaceutically acceptable salt thereof.

In another particular embodiment of the invention, the compound of formula (I) is 3-[(3-fluorophenyl)sulfonyl]-8-(1-piperazinyl)quinoline, or a pharmaceutically acceptable salt thereof.

3-Phenylsulfonyl-8-piperazin-1-yl-quinoline and 3-[(3-fluorophenyl)sulfonyl]-8-(1-piperazinyl)quinoline are known to have 5-HT₆ receptor antagonist activity and are disclosed in WO 03/080580, see examples 2, 7, 16, 51 and 52.

In another embodiment of the invention, a method of treatment of inflammatory pain in mammals is provided, which method comprises the administration to the mammal in need of such treatment, an effective amount of a compound of formula (I), or a pharmaceutically acceptable salt thereof.

The cause of such inflammatory pain may be osteoarthritis or rheumatoid arthritis. There is therefore provided in one embodiment of the invention, a method of treatment of chronic articular pain associated with osteoarthritis or rheumatoid arthritis in mammals, which method comprises the administration to the mammal in need of such treatment, an effective amount of a compound of formula (I), or a pharmaceutically acceptable salt thereof.

In another embodiment of the invention, a method of treatment of neuropathic pain in mammals is provided, which method comprises the administration to the mammal in need of such treatment, an effective amount of a compound of formula (I), or a pharmaceutically acceptable salt thereof.

In another embodiment of the invention, a method of treatment of visceral pain in mammals is provided, which method comprises the administration to the mammal in need of such treatment, an effective amount of a compound of formula (I), or a pharmaceutically acceptable salt thereof. In one embodiment of the invention, the visceral pain is associated with irritable bowel syndrome.

In another embodiment of the invention, a method of treatment of irritable bowel syndrome in mammals is provided, which method comprises the administration to the mammal in need of such treatment, an effective amount of a compound of formula (I), or a pharmaceutically acceptable salt thereof. In one embodiment of the invention, the mammal in need of such treatment is human and female.

In another embodiment of the invention, a method of treatment of headache is provided, which method comprises the administration to the mammal in need of such treatment, an effective amount of a compound of formula (I), or a pharmaceutically acceptable salt thereof.

Other aspects and optional features of the invention are set forth in the appended claims.

The free base of 3-phenylsulfonyl-8-piperazin-1-yl-quinoline and the hydrochloride salt of 3-[(3-fluorophenyl)sulfonyl]-8-(1-piperazinyl)quinoline are referred to throughout this application as SB742457 and SB792988A respectively. In all experiments where SB742457 was used, SB742457 was used as the free base polymorphic form II (see WO 03/080580, Example 52).

As used herein, the term "pain" refers to any unpleasant sensation that is perceived by the individual and includes, acute pain, chronic pain, somatic pain (originating from ligaments, tendons, bones, blood vessels or nerves), chronic articular pain, musculoskeletal pain, neuropathic pain, inflammatory pain, visceral pain, pain associated with cancer, pain associated with migraine, tension headache and cluster headaches, pain associated with functional bowel disorders, lower back and neck pain, pain associated with sprains and strains, sympathetically maintained pain; myositis, pain associated with influenza or other viral infections such as the common cold, pain associated with rheumatic fever, pain associated with myocardial ischemia, post operative pain, cancer chemotherapy, headache, toothache and dysmenorrhea.

As used herein, the term "inflammatory pain" refers to any kind of pain that results from the inflammation of bodily tissues and includes, inflammation resulting from soft tissue damage or infection.

As used herein, the term "neuropathic pain" refers to any kind of pain that results from injury or disease to the nerve tissue itself and includes : diabetic neuropathy, sciatica, non-specific lower back pain, trigeminal neuralgia, multiple sclerosis pain, fibromyalgia, HIV-related neuropathy, post-herpetic neuralgia, trigeminal neuralgia, and pain resulting from physical trauma, imputation, phantom limb syndrome, spinal surgery, cancer, toxins or chronic inflammatory conditions. In addition, neuropathic pain conditions include pain associated with normally non-painful sensations such as "pins and needles" (paraesthesias and dysesthesias), increased sensitivity to touch (hyperesthesia), painful sensation following innocuous stimulation (dynamic, static, thermal or cold allodynia), increased sensitivity to noxious stimuli (thermal, cold, mechanical hyperalgesia), continuing pain sensation after removal of the stimulation (hyperpathia) or an absence of or deficit in selective sensory pathways (hypoalgesia).

As used herein, the term "visceral pain" refers to any kind of pain that originates from the body's internal cavities or organs and includes pain that originates from the intestines.

As used herein, the term "Irritable Bowel Syndrome" (IBS) is defined according to the Rome III diagnostic criteria where the criteria are fulfilled for the last 3 months with symptom onset at least 6 months prior to diagnosis. The Rome III diagnostic criteria for IBS are as follows:
- Recurrent abdominal pain or discomfort (where discomfort means an uncomfortable sensation not described as pain) at least 3 days per month in the last 3 months associated with 2 or more of the following:
   1. Improvement with defecation;
   2. Onset associated with a change in frequency of stool;
   3. Onset associated with a change in form (appearance) of stool.

Other symptoms that are not essential but support the diagnosis of IBS include:
- Abnormal stool frequency (greater than 3 bowel movements/day or less than 3 bowel movements/week);
- Abnormal stool form (lumpy/hard or loose watery stool);
- Abnormal stool passage (straining, urgency, or feeling of incomplete bowel movement):
- Passage of mucous;
- Bloating or feeling of abdominal distension.

As used herein, the term "headache" refers to any unpleasant sensation that is localised to the individual head and includes, migraine, tension headache and cluster headaches.

### List of Figures

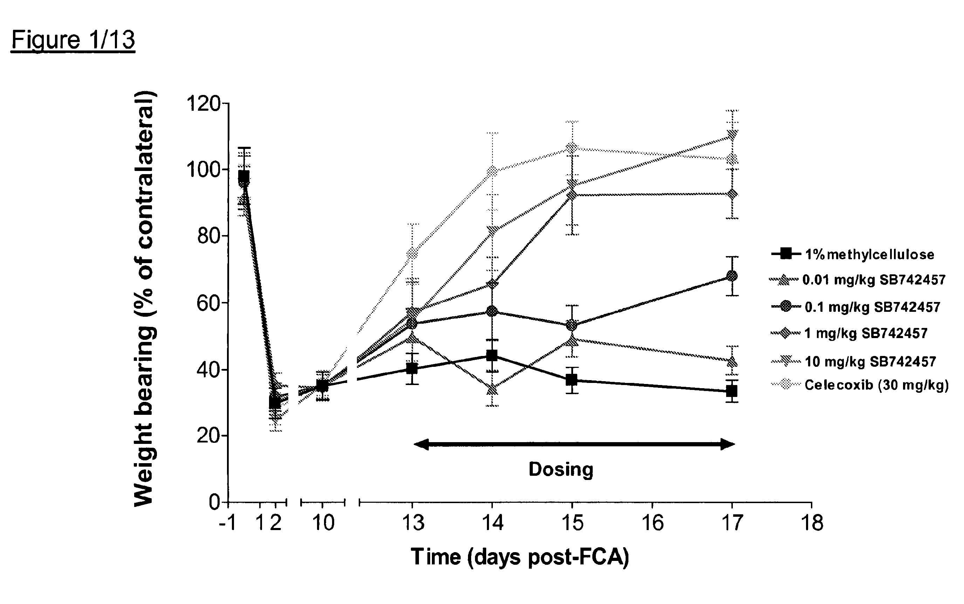

Figures 1 shows the effect of SB742457 versus celecoxib administered orally on hypersensitivity in the rat joint pain model of chronic inflammatory pain.
   Vehicle (1% methylcellulose), SB742457 and celecoxib were administered by oral gavage over the time course indicated following 150 µl FCA intra-articular injection into the left knee. Hind paw weight bearing data was calculated and expressed as the percentage of the contralateral paw. 10 animals used per group.
   All data are expressed as mean ± s.e.m. Statistical analysis was carried out using ANOVA followed by Duncan's post-hoc comparisons (p<0.05 considered significant, see Table 2).
Figure 2 shows the Area Under the Curve (AUC) for vehicle, each dose of SB742457 and for 30mg/kg celecoxib, as plotted in Figure 1.
   All data are expressed as mean ± s.e.m. *** denotes p<0.001 following statistical comparison of vehicle versus SB742457 and celecoxib treated animals using ANOVA and Duncan's post-hoc comparisons (p<0.05 considered significant).
Figure 3 shows the effect of SB792988A versus celecoxib administered orally on hypersensitivity in the rat joint pain model of chronic inflammatory pain.
   Vehicle (1% methylcellulose), SB792988A and celecoxib were administered by oral gavage over the time course indicated following 150 µl FCA intra-articular injection into the left knee. Hind paw weight bearing data was calculated and expressed as the percentage of the contralateral paw. 10 animals used per group.
   All data are expressed as mean ± s.e.m. Statistical analysis was carried out using ANOVA followed by Duncan's post-hoc comparisons (p<0.05 considered significant, see Table 3).

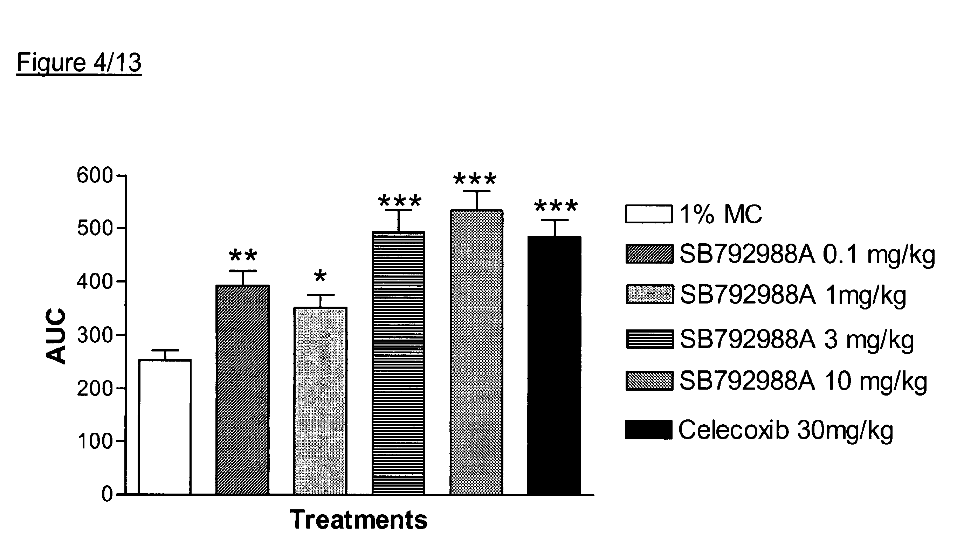

Figure 4 shows the Area Under the Curve (AUC) for vehicle, each dose of SB792988A and for 30mg/kg celecoxib, as plotted in Figure 1.
   All data are expressed as mean ± s.e.m. * denotes p<0.05, ** denotes p<0.01 and *** denotes p<0.001 following statistical comparison of vehicle versus SB792988A and celecoxib treated animals using ANOVA and Duncan's post-hoc comparisons (p<0.05 considered significant).

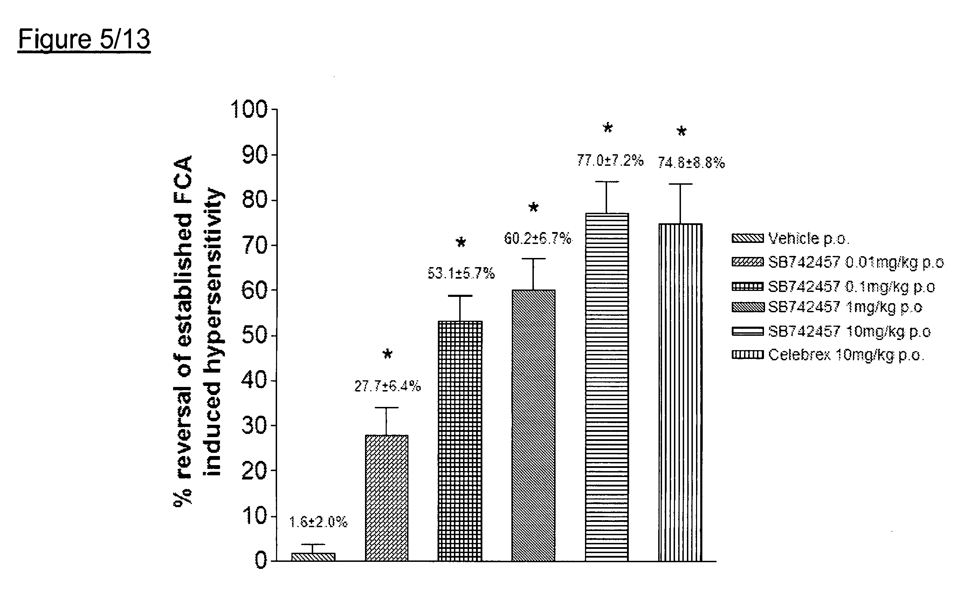

Figure 5 shows the effect of SB742457 administered orally on hypersensitivity in the rat FCA induced hypersensitivity model.
   Vehicle (1% methylcellulose), SB742457, and celecoxib were administered by oral gavage 24 hours following 100 µl FCA intraplantar injection into the left hind paw. Hind paw weight bearing data was calculated and expressed as the percentage of the contralateral paw. 7 animals used per group.
   All data are expressed as mean ± s.e.m. * denotes p<0.05 following statistical comparison of vehicle versus, SB742457 and celecoxib treated animals using ANOVA and Fischer LSD test (p<0.05 considered significant).

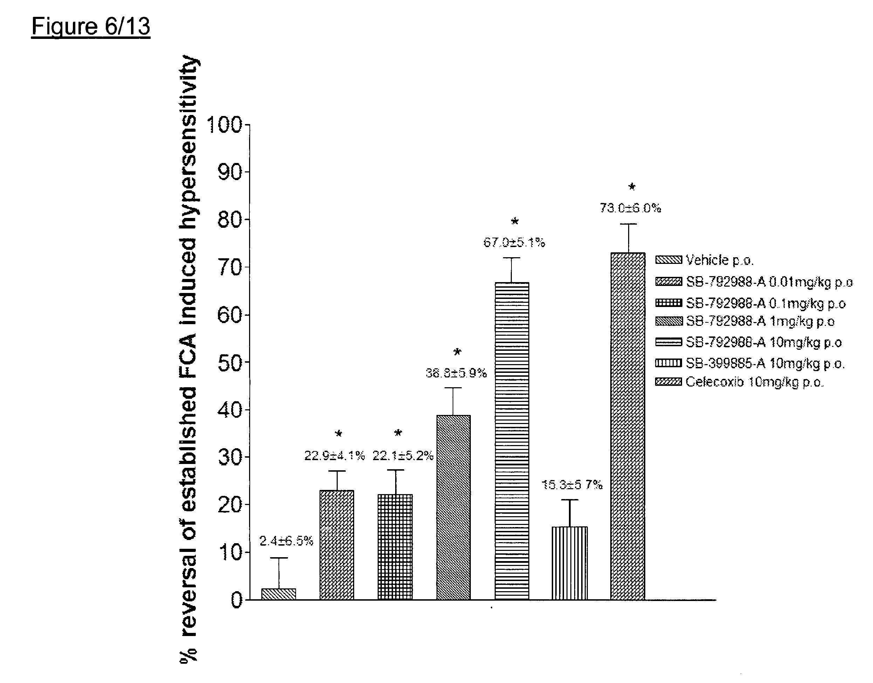

Figure 6 shows the effect of SB792988A versus SB399885A (another compound known to have 5-HT₆ receptor antagonist activity, see WO 02/18358, Example 2) administered orally on hypersensitivity in the rat FCA induced hypersensitivity model. Vehicle (1% methylcellulose), SB792988A, SB399885A and celecoxib were administered by oral gavage 24 hours following 100 µl FCA intraplantar injection into the left hind paw. Hind paw weight bearing data was calculated and expressed as the percentage of the contralateral paw. 7 animals used per group.
   All data are expressed as mean ± s.e.m. * denotes p<0.05 following statistical comparison of vehicle versus SB792988A, SB399885 and celecoxib treated animals using ANOVA and Fischer LSD test (p<0.05 considered significant).

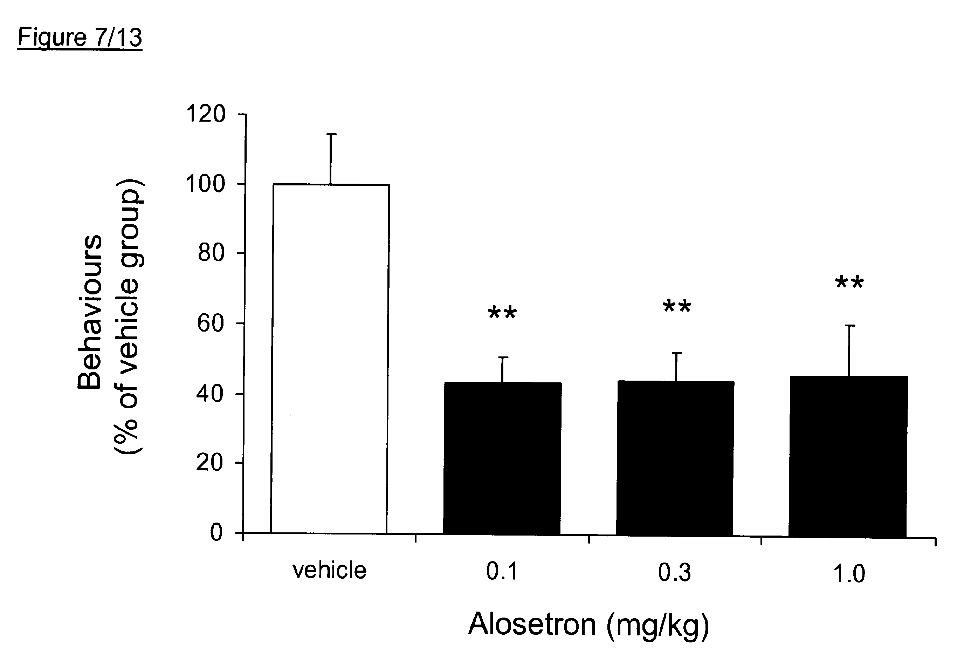

Figure 7 shows the effect of alosetron administered sub cutaneously on pain behaviour produced in response to intra rectal injection of mustard oil in male Sprague Dawley rats.
   Vehicle (saline) and alosetron administered by sub cutaneous injection 15 minutes prior to intra rectal injection of mustard oil (3% mustard oil, 70% ethanol in saline). 10 animals used per group. Pain behaviours were counted for 25 minutes following injection of mustard oil and expressed as a percentage of the vehicle treated response.
   All data are expressed as mean ± s.e.m. ** denotes p<0.01 following statistical comparison of vehicle versus Alosetron treated animals using a one way ANOVA and Dunnett's post hoc test.

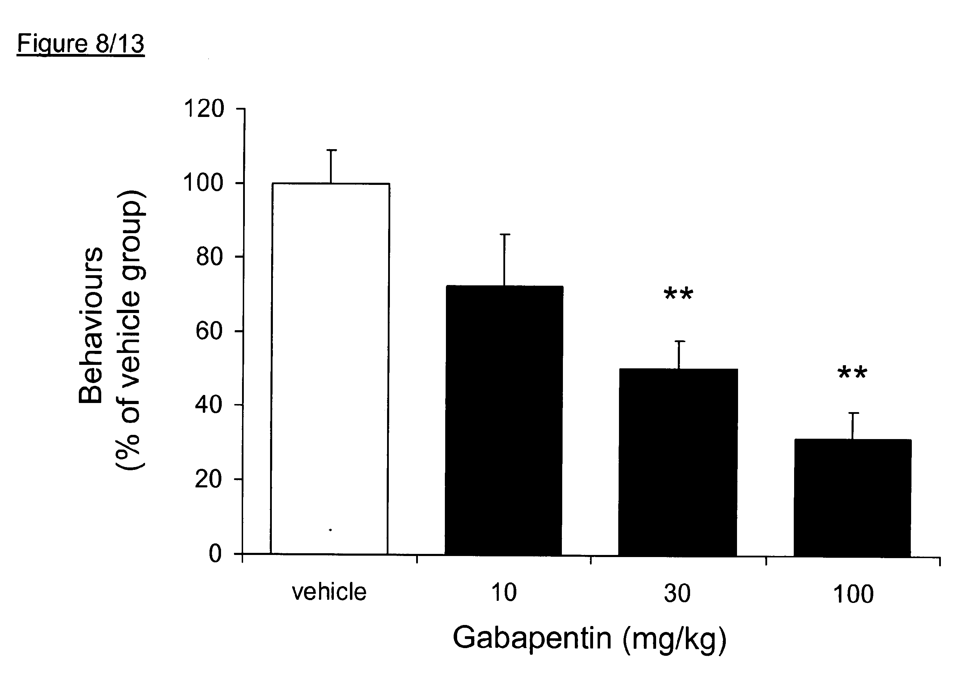

Figure 8 shows the effect of gabapentin administered subcutaneously on pain behaviour produced in response to intra rectal injection of mustard oil in male Sprague Dawley rats.
   Vehicle (10% 1-Methyl-2-pyrrolidone in saline) and gabapentin administered by subcutaneous injection 15 minutes prior to intra rectal injection of mustard oil (3% mustard oil, 70% ethanol in saline). 10 animals used per group. Pain behaviours were counted for 25 minutes following injection of mustard oil and expressed as a percentage of the vehicle treated response.
   All data are expressed as mean ± s.e.m. ** denotes p<0.01 following statistical comparison of vehicle versus gabapentin treated animals using a one way ANOVA and Dunnett's post hoc test.

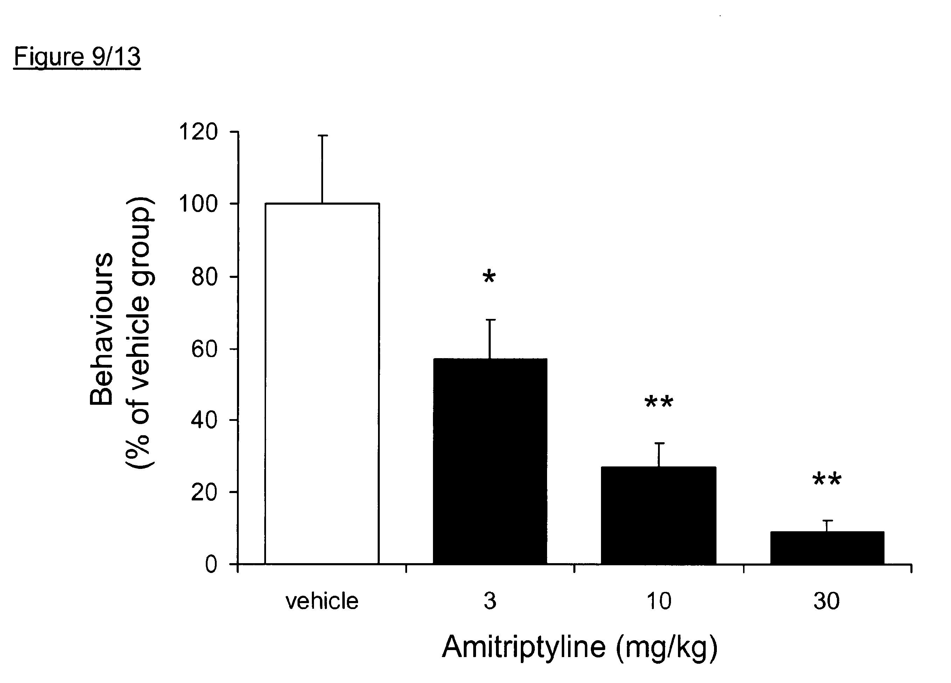

Figure 9 shows the effect of amitriptyline administered subcutaneously on pain behaviour produced in response to intra rectal injection of mustard oil in male Sprague Dawley rats.
   Vehicle (saline) and amitriptyline administered by subcutaneous injection 15 minutes prior to intra rectal injection of mustard oil (3% mustard oil, 70% ethanol in saline). 10 animals used per group. Pain behaviours were counted for 25 minutes following injection of mustard oil and expressed as a percentage of the vehicle treated response.
   All data are expressed as mean ± s.e.m. * denotes p<0.05, ** denotes p<0.01 following statistical comparison of vehicle versus amitriptyline treated animals using a one way ANOVA and Fischer's post hoc test.

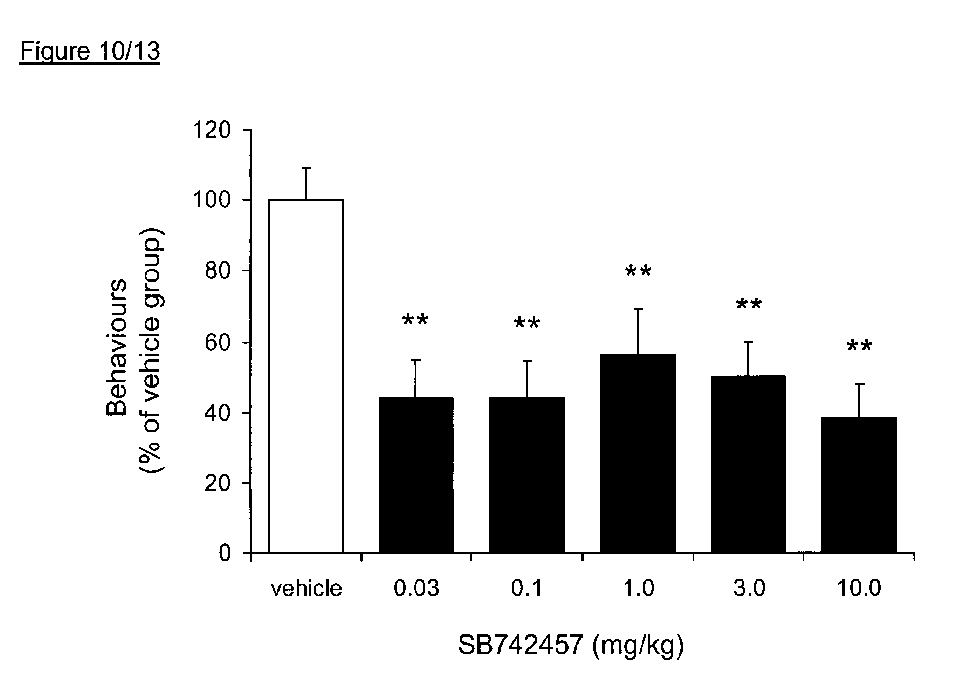

Figure 10 shows the effect of SB742457 administered orally on pain behaviour produced in response to intra rectal injection of mustard oil in male Sprague Dawley rats.
   Vehicle (1% methyl cellulose in water) and SB742457 administered by oral gavage 60 minutes prior to intra rectal injection of mustard oil (3% mustard oil, 70% ethanol in saline). 10-20 animals used per group. Pain behaviours were counted for 25 minutes following injection of mustard oil and expressed as a percentage of the vehicle treated response.
   ** denotes p<0.01 following statistical comparison of vehicle versus SB742457 treated animals using a one way ANOVA and Dunnett's post hoc test.

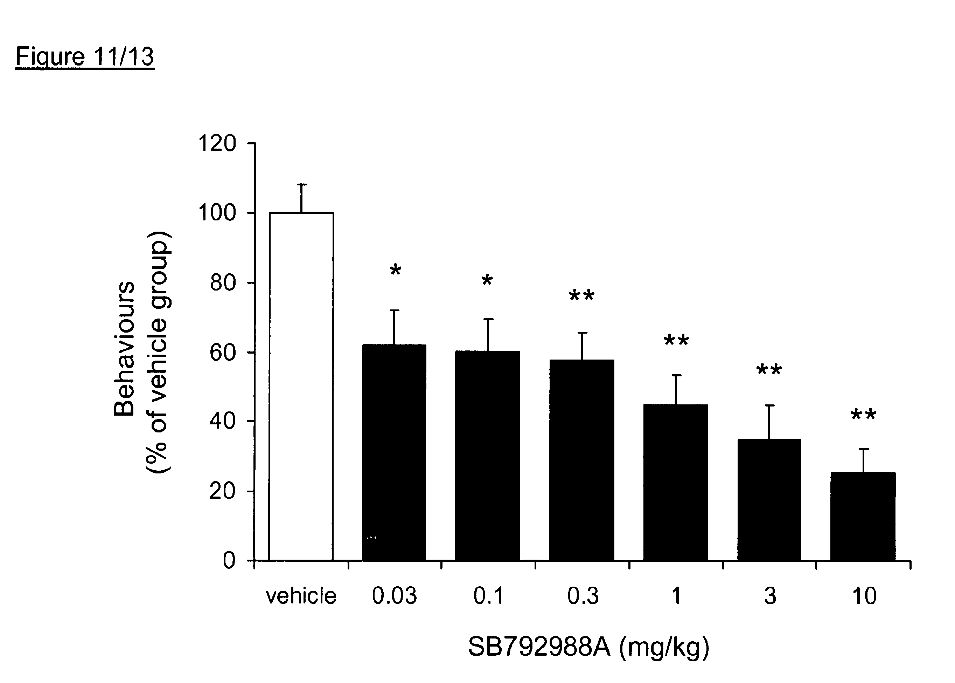

Figure 11 shows the effect of SB792988A administered orally on pain behaviour produced in response to intra rectal injection of mustard oil in male Sprague Dawley rats.
   Vehicle (1% methyl cellulose in water) and SB792988A administered by oral gavage 60 minutes prior to intra rectal injection of mustard oil (3% mustard oil, 70% ethanol in saline). 10-20 animals used per group. Pain behaviours were counted for 25 minutes following injection of mustard oil and expressed as a percentage of the vehicle treated response.
   All data are expressed as mean ± s.e.m. * denotes p<0.05, ** denotes p<0.01 following statistical comparison of vehicle versus SB792988 treated animals using a one way ANOVA and Dunnett's post hoc test.

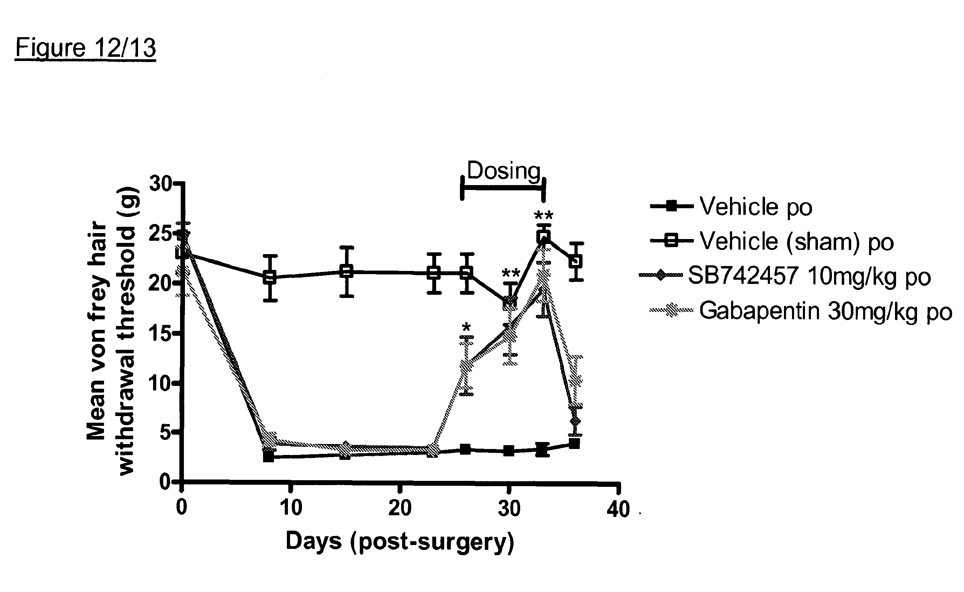

Figure 12 shows the effect of SB742457 administered orally on pain behaviour produced in response to the CCl model of neuropathic pain in rats. The sciatic nerve in the left leg of the rat was exposed at mid thigh level and the wound was closed and secured with staples. The Sham operated animals underwent the same surgical technique except that the nerve was not ligated. The presence of mechanical (tactile) allodynia was assessed using the manual application of Von Frey hair monofilaments and neuropathy was maintained as a stable baseline until day 23 post-surgery, when the animals were randomised and then chronically dosed with either SB742457 (10mg/kg b.i.d. po), gabapentin (30mg/kg b.i.d. po) or vehicle (1% methylcellulose; b.i.d. po) for 8 days (days 26-33 post-surgery).
   All data are expressed as mean ± s.e.m. * denotes p<0.1, ** denotes p<0.01 following statistical comparison between SB742457 and vehicle treated CCI animals using a one-way ANOVA (P<0.05 considered significant).

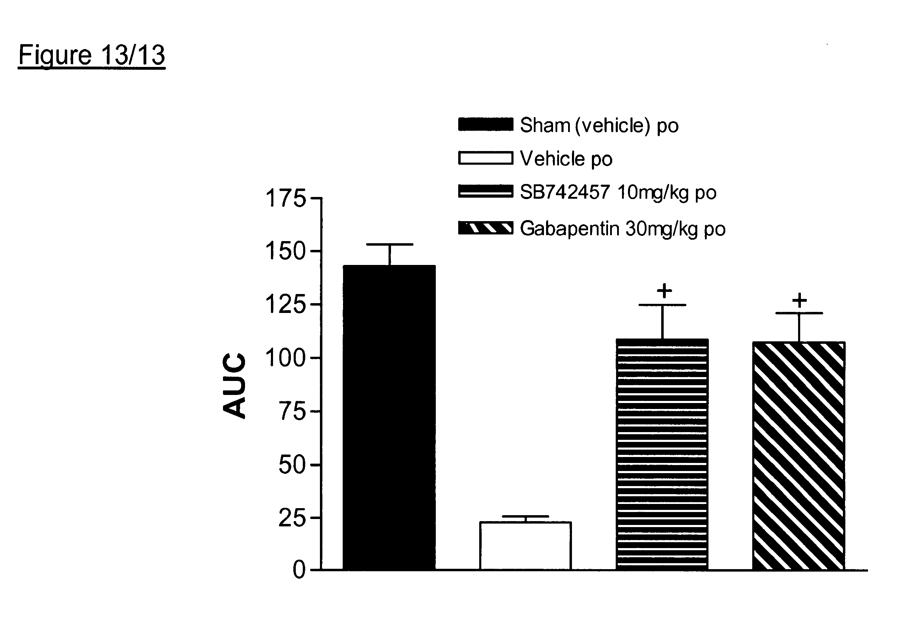

Figure 13 shows the Area Under the Curve (AUC) for vehicle, each dose of SB742457 (10mg/kg b.i.d. po), gabapentin (30mg/kg b.i.d. po) or vehicle (1% methylcellulose; b.i.d. po), as plotted in Figure 12.
   All data are expressed as mean ± s.e.m. ⁺ denotes p<0.05 following statistical comparison between SB742457 and vehicle CCI and Sham groups. AUC calculations were performed within Excel and statistical analysis was carried out using a one-way ANOVA followed by Fischer LSD post-hoc test (Statistica Version 6) to compare the vehicle treated CCI and Sham groups with the drug treated groups (P<0.05 considered significant).

### Description

The rat FCA models of inflammatory pain were validated by using the NSAID celecoxib as a positive control when testing the effect of 3-phenylsulfonyl-8-piperazin-1-yl-quinoline (SB742457) and the hydrochloride salt of 3-[(3-fluorophenyl)sulfonyl]-8-(1-piperazinyl)quinoline (SB792988A). Celecoxib reversed the hypersensitivity to chronic joint pain produced by intra-articular injection of FCA compared to vehicle when used in the rat model of chronic inflammatory pain. Celecoxib also reversed hypersensitivity induced by the intraplantar injection of FCA.

These observations thus validate these models as useful predictors of compounds likely to have an effect on inflammatory pain in any mammal, including humans.

When used in the rat FCA models of inflammatory pain, SB742457 and SB792988A produced significant reductions in hypersensitivity to chronic joint pain and also in hypersensitivity induced by intraplantar FCA injection, comparable to that observed with the positive control celecoxib. By comparison with celecoxib in the rat FCA models of inflammatory pain, it is thus likely that SB742457 and SB792988A will have beneficial effects on inflammatory pain in other mammals, including humans.

The rat intra rectal mustard oil model of visceral pain was validated by pre-treatment of rats with alosetron, gabapentin or amitriptyline, which are effective at treating IBS and the visceral pain associated with IBS in humans. Pre-treatment with alosetron, gabapentin or amitriptyline produced a significant reduction in the number of visceral pain related behaviours compared to vehicle-treated animals, thus validating this model as a useful predictor of compounds likely to have an effect on visceral pain and the visceral pain associated with IBS in humans.

When used in the rat intra rectal mustard oil model of visceral pain, SB742457 and SB792988A produced significant reductions in visceral pain related behaviours, comparable to those observed with alosetron, gabapentin or amitriptyline. By comparison with alosetron, gabapentin and amitriptyline, it is thus likely that SB742457 and SB792988A will have beneficial effects on visceral pain and visceral pain associated with IBS in other mammals, including humans.

The Chronic Constriction Injury (CCI) model is a model of nerve damage-induced neuropathic pain in rats. The CCI model is believed to involve mechanisms, which contribute to neuropathic pain such as central (spinal cord) sensitisation (Kajander, KC *et al.* (1990) Peptides, **11**, 719-728; Wakisaka, S *et al*. (1992) Brain Research **598** (**1-2**), 349-352; Mao, J *et al*. (1993) J. Neurophysiol., **70**, 470-481). It is also believed that a peripheral component is involved in the CCI model due to inflammation arising at the site of nerve ligation (Basbaum, Al *et al.* (1991) Pain **47**, 359-367). SB742457 significantly reversed CCI-induced mechanical allodynia within 1 hr of dosing, which was maintained for the duration of the dosing period. It is therefore likely that SB742457 will have beneficial effects on neuropathic pain in other mammals, including humans.

Compounds of formula (I) may be synthesised by reacting a compound of formula (II) 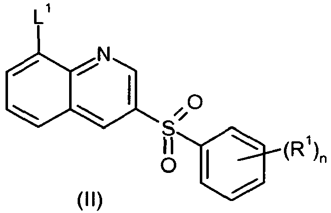 with a compound of formula (III) 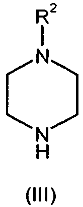 wherein R¹ and n are as defined above, R² represents an *N*-protecting group or hydrogen and L¹ represents a suitable leaving group, such as a halogen atom (e.g. chlorine, iodine, or, when R² is H, fluorine) or a trifluoromethylsulfonyloxy group, and thereafter as necessary removing an R² *N*-protecting group. The *N*-protecting group used may be any conventional group e.g. *t*-butyloxycarbonyl (Boc) or benzyloxycarbonyl. Further *N*-protecting groups which may be used include methyl.

The above process may be performed in the presence of a palladium, nickel or copper catalyst, for example a mixture of a palladium source such as Pd₂(dba)₃ and a suitable ligand such as (R)-, (S)- or (±)-2,2'-bis(diphenylphosphino)-1,1'-binaphthyl (BINAP) or (2-dicyclohexylphosphanylphenyl)-dimethylamine or 1,1'-bis-diphenylphosphinoferrocene, together with a suitable base such as sodium *t-*butoxide, in an inert solvent such as 1,4-dioxane.

Where L¹ is fluorine, the above reaction may be carried out in the presence of a suitable base such as potassium carbonate, a suitable solvent such as n-propanol and at a suitable temperature such as 100°C.

Compounds of formula (II) may be formed by reacting a compound of formula (IV): 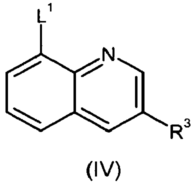 with the sodium salt of a compound of formula (V): 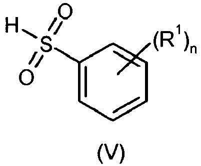 wherein L¹ represents fluorine or chlorine, R¹ and n are as defined above and R³ represents iodine or bromine; in the presence of a diamine ligand such as ethylenediamine-tetraacetate (EDTA) or N,N'-dimethylethylenediamine, a metal catalyst such as copper iodide (Cul), a base such as diisopropylethylamine, and a polar aprotic solvent such as dimethylsulfoxide, dimethylformamide or hexamethylphosphorotriamide.

Compounds of formula (II) may also be formed by oxidising a compound of formula (VI): 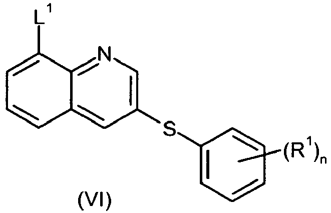 with a suitable oxidant such as monomagnesium peroxyphthalate, 3-chloroperbenzoic acid, peracetic acid or potassium monopersulfate.

Compounds of formula (VI) may be formed by reacting a compound of formula (VII): 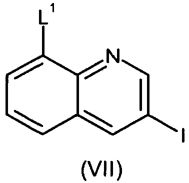 with a compound of formula (VIII): 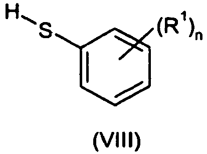 wherein L¹, R¹ and n are as defined above, in the presence of a base such as sodium hydride or potassium phosphate in a suitable solvent such as anhydrous *N*,*N-*dimethylformamide or ethylene glycol, optionally in the presence of a copper (I) iodide catalyst.

Compounds of formula (I) may in some circumstances form acid addition salts, for example the hydrochloride salt of 3-[(3-fluorophenyl)sulfonyl]-8-(1-piperazinyl)quinoline (SB792988A). It will be appreciated that for use in medicine compounds of formula (I) may be used as salts, in which case the salts should be pharmaceutically acceptable. Pharmaceutically acceptable salts include those described by Berge, Bighley and Monkhouse , J. Pharm. Sci., 1977, 66, 1-19. Salts may be prepared from pharmaceutically acceptable acids, including inorganic and organic acids. Such acids include acetic, benzenesulfonic, benzoic, camphorsulfonic, citric, ethanesulfonic, fumaric, gluconic, glutamic, hydrobromic, hydrochloric, lactic, maleic, malic, mandelic, methanesulfonic, mucic, nitric, pamoic, pantothenic, phosphoric, succinic, sulfuric, tartaric, p-toluenesulfonic acid.

Examples of pharmaceutically acceptable salts include those formed from maleic, fumaric, benzoic, ascorbic, pamoic, succinic, hydrochloric, sulfuric, bismethylenesalicylic, methanesulfonic, ethanedisulfonic, propionic, tartaric, salicylic, citric, gluconic, aspartic, stearic, palmitic, itaconic, glycolic, p-aminobenzoic, glutamic, benzenesulfonic, cyclohexylsulfamic, phosphoric and nitric acids.

The compounds of formula (I) may be prepared in crystalline or non-crystalline form, and, if crystalline, may optionally be solvated, e.g. as the hydrate. This invention includes within its scope stoichiometric solvates (e.g. hydrates) as well as compounds containing variable amounts of solvent (e.g. water).

A pharmaceutical composition of the invention, which may be prepared by admixture, suitably at ambient temperature and atmospheric pressure, is usually adapted for oral, parenteral or rectal administration and, as such, may be in the form of tablets, capsules, oral liquid preparations, powders, granules, lozenges, reconstitutable powders, injectable or infusable solutions or suspensions or suppositories. Orally administrable compositions are generally preferred.

Tablets and capsules for oral administration may be in unit dose form, and may contain conventional excipients, such as binding agents, fillers, tabletting lubricants, disintegrants and acceptable wetting agents. The tablets may be coated according to methods well known in normal pharmaceutical practice.

Oral liquid preparations may be in the form of, for example, aqueous or oily suspension, solutions, emulsions, syrups or elixirs, or may be in the form of a dry product for reconstitution with water or other suitable vehicle before use. Such liquid preparations may contain conventional additives such as suspending agents, emulsifying agents, non-aqueous vehicles (which may include edible oils), preservatives, and, if desired, conventional flavourings or colourants.

For parenteral administration, fluid unit dosage forms are prepared utilising a compound of the invention or pharmaceutically acceptable salt thereof and a sterile vehicle. The compound, depending on the vehicle and concentration used, can be either suspended or dissolved in the vehicle. In preparing solutions, the compound can be dissolved for injection and filter sterilised before filling into a suitable vial or ampoule and sealing. Advantageously, adjuvants such as a local anaesthetic, preservatives and buffering agents are dissolved in the vehicle. To enhance the stability, the composition can be frozen after filling into the vial and the water removed under vacuum. Parenteral suspensions are prepared in substantially the same manner, except that the compound is suspended in the vehicle instead of being dissolved, and sterilization cannot be accomplished by filtration. The compound can be sterilised by exposure to ethylene oxide before suspension in a sterile vehicle. Advantageously, a surfactant or wetting agent is included in the composition to facilitate uniform distribution of the compound.

The composition may contain from 0.1 % to 99% by weight, preferably from 10 to 60% by weight, of the active material, depending on the method of administration.

The dose of the compound used in the treatment of the disorders mentioned herein will vary in the usual way with the seriousness of the disorders, the weight of the sufferer, and other similar factors. However, as a general guide suitable unit doses may be 0.05 to 1000 mg, more suitably 0.05 to 200 mg, for example 5 to 35 mg; and such unit doses will preferably be administered once a day, although administration more than once a day may be required; and such therapy may extend for a number of weeks, months or even years. In addition, such therapy could be given on demand, prophylactically, or continuously over a period of time until the patient no longer requires treatment. An example of a suitable dosing regimen would be a 5, 15 or 35 mg once daily dosing given prophylactically, or continuously over a period of time until the patient no longer requires treatment.

### Clinical Indications

It is believed that compounds of formula (I), or a pharmaceutically acceptable salt thereof, may be useful in the treatment of pain, including acute pain, chronic pain, somatic pain (originating from ligaments, tendons, bones, blood vessels or nerves), chronic articular pain, musculoskeletal pain, neuropathic pain, inflammatory pain, visceral pain, pain associated with cancer, pain associated with migraine, tension headache and cluster headaches, pain associated with functional bowel disorders, lower back and neck pain, pain associated with sprains and strains, sympathetically maintained pain; myositis, pain associated with influenza or other viral infections such as the common cold, pain associated with rheumatic fever, pain associated with myocardial ischemia, post operative pain, cancer chemotherapy, headache, toothache and dysmenorrhea.

Chronic articular pain conditions include rheumatoid arthritis, osteoarthritis, rheumatoid spondylitis, gouty arthritis and juvenile arthritis.

Pain associated with functional bowel disorders includes non-ulcer dyspepsia, non-cardiac chest pain and irritable bowel syndrome.

Neuropathic pain syndromes include: diabetic neuropathy, sciatica, non-specific lower back pain, trigeminal neuralgia, multiple sclerosis pain, fibromyalgia, HIV-related neuropathy, post-herpetic neuralgia, trigeminal neuralgia, and pain resulting from physical trauma, amputation, phantom limb syndrome, spinal surgery, cancer, toxins or chronic inflammatory conditions. In addition, neuropathic pain conditions include pain associated with normally non-painful sensations such as "pins and needles" (paraesthesias and dysesthesias), increased sensitivity to touch (hyperesthesia), painful sensation following innocuous stimulation (dynamic, static, thermal or cold allodynia), increased sensitivity to noxious stimuli (thermal, cold, mechanical hyperalgesia), continuing pain sensation after removal of the stimulation (hyperpathia) or an absence of or deficit in selective sensory pathways (hypoalgesia).

Other conditions which could potentially be treated by compounds of formula (I), or a pharmaceutically acceptable salt thereof but which are not part of the invention include fever, inflammation, immunological diseases, abnormal platelet function diseases (e.g. occlusive vascular diseases), impotence or erectile dysfunction; bone disease characterised by abnormal bone metabolism or resorbtion; hemodynamic side effects of non-steroidal antiinflammatory drugs (NSAID's) and cyclooxygenase-2 (COX-2) inhibitors, cardiovascular diseases; neurodegenerative diseases and neurodegeneration, neurodegeneration following trauma, tinnitus, dependence on a dependence-inducing agent such as opiods (e.g. morphine), CNS depressants (e.g. ethanol), psychostimulants (e.g. cocaine) and nicotine; complications of Type I diabetes, kidney dysfunction, liver dysfunction (e.g. hepatitis, cirrhosis), gastrointestinal dysfunction (e.g. diarrhoea), colon cancer, overactive bladder and urge incontinence. Depression and alcoholism could potentially also be treated by compounds of formula (I), or a pharmaceutically acceptable salt thereof.

Inflammatory conditions include skin conditions (e.g. sunburn, burns, eczema, dermatitis, allergic dermatitis, psoriasis), meningitis, ophthalmic diseases such as glaucoma, retinitis, retinopathies, uveitis and of acute injury to the eye tissue (e.g. conjunctivitis), inflammatory lung disorders (e.g. asthma, bronchitis, emphysema, allergic rhinitis, respiratory distress syndrome, pigeon fancier's disease, farmer's lung, chronic obstructive pulmonary disease (COPD), airways hyperresponsiveness); gastrointestinal tract disorders (e.g. aphthous ulcer, Crohn's disease, atopic gastritis, gastritis varialoforme, ulcerative colitis, coeliac disease, regional ileitis, irritable bowel syndrome, inflammatory bowel disease, gastrointestinal reflux disease); organ transplantation and other conditions with an inflammatory component such as vascular disease, migraine, periarteritis nodosa, thyroiditis, aplastic anaemia, Hodgkin's disease, sclerodoma, myaesthenia gravis, multiple sclerosis, sorcoidosis, nephrotic syndrome, Bechet's syndrome, gingivitis, myocardial ischemia, pyrexia, systemic lupus erythematosus, polymyositis, tendinitis, bursitis, and Sjogren's syndrome.

It is to be understood that reference to treatment includes both treatment of established symptoms and prophylactic treatment, unless explicitly stated otherwise.

### Examples

### Example 1: Synthesis of 3-phenylsulfoyl-8-piperazin-1-yl-quinoline

The following Example illustrates the preparation of 3-phenylsulfonyl-8-piperazin-1-yl-quinoline, but is not intended to be limiting.

Proton Magnetic Resonance (NMR) spectra were recorded on a Bruker instrument at 400 MHz. Chemical shifts are reported in ppm (d) using tetramethylsilane as internal standard. Splitting patterns are designated as s, singlet; d, doublet; t, triplet; q, quartet; m, multiplet; br, broad.

### Intermediate 1: 8-Fluoro-3-iodoquinoline

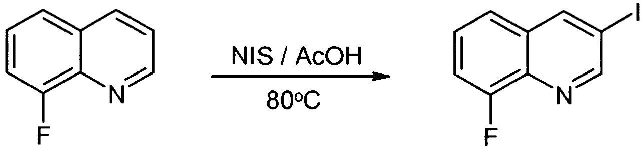

N-Iodosuccinimide (NIS) (68.56 g, 305.81 mmol) was added to a solution of 8-fluoroquinoline (30 g, 203.87 mmol) in glacial acetic acid (AcOH) (129 ml). The mixture was stirred and heated to 80°C, under N₂ in a 250 mL CLR (Controlled Laboratory Reactor).

After 24 hrs Na₂SO₃ (15 g) was added to the flask with H₂O (63 ml) and the solution was stirred, whilst being maintained at 80°C for 1 hour to quench the remaining iodine. After an hour the reaction was allowed to cool from 80°C to 22°C over 30 minutes. Once 22°C had been reached the crystals were filtered off under vacuum and washed with 2:1 AcOH/H₂O (60 ml) and H₂O (3 x 180 mL) and the crystals were pulled dry. The crystals were dried in an oven which was connected to an oil pump at 50°C under reduced pressure.

The cake was removed from the oven to afford the title compound as a pale brown solid (38.63 g, 66%).

### Intermediate 1 (alternative process): 8-Fluoro-3-iodoguinoline

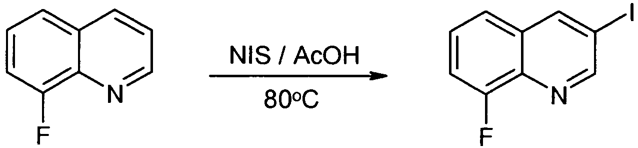

N-lodosuccinimide (NIS) (229.0 g, 1.018 mol) was added to a stirred solution of 8-fluoroquinoline (100.0 g, 0.68 mol) in glacial acetic acid (AcOH) (430 ml). 8-Fluoroquinoline may be obtained from Orgasynth (www.orgasynth.com). The mixture was heated to circa 80°C under nitrogen. After 23.5 hr sodium sulphite (50.0 g, 0.397 mol) and water (210 ml) were added and the mixture reheated to circa 80°C. After 1.5 hr the mixture was allowed to cool to circa 60-65°C and seeded with 8-fluoro-3-iodoquinoline (100 mg). The product soon crystallised and the stirred slurry was allowed to cool over 1.5 hr to ambient temperature. After 1.25 hr the product was collected by vacuum filtration. The bed was washed with 1:1 acetic acid / water (2 x 300 ml) and water (2 x 300 ml). The bed was pulled dry for 5 min and the material used without further processing.

A sample of the material was dried *in vacuo* at 40-45°C, to afford the desired product in 75% yield.
¹H NMR, D₄ MeOH, 400 MHz
7.50 ppm (1H, ddd, *J* 1.5, 7.5 & 11.0 Hz), 7.58 ppm (1H, dt, *J* 5 & 8 Hz), 7.64 ppm (1H, dd, *J* 1.0 & 8.5 Hz), 8.78 ppm (1H. t, *J* 1.5 Hz), 8.99 ppm (1H, d, *J* 2.0 Hz)

### Intermediate 2: 8-Fluoro-3-phenylsulfonylquinoline

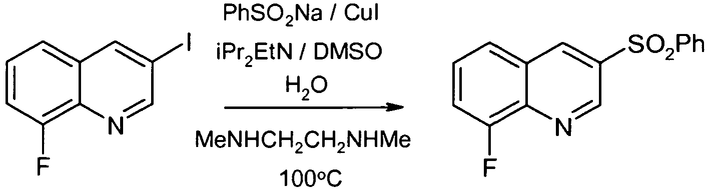

Copper iodide (Cul) (0.7 g) was added to a stirred solution of dimethylsulfoxide (50 ml) and 85% N,N'-dimethylethylenediamine (0.92 ml). The mixture was stirred at ambient temperature for 5 min to effect solution. Water (20 ml) was added (exothermic, contents increased to 40°C) and contents maintained at 40-50°C. Diisopropylethylamine (6.4 ml), benzenesulfinic acid sodium salt (12.0 g) and 8-fluoro-3-iodoquinoline (10.0 g) were added sequentially and the resulting slurry heated under nitrogen to 100°C, then maintained at 100°C for 12 hr. After which time the reaction mixture was cooled to 20°C over 1 hour then aged for 5 hr at 20°C. The product was collected by vacuum filtration and the cake was washed with 5:2 v/v dimethylsulfoxide - water (2 x 10 ml) and water (2 x 20 ml). The bed was pulled dry and the product dried *in vacuo* at 50°C, to give the title compound, 8.04 g, 76% yield. ¹H NMR, CDCl₃, 400 MHz
7.54-7.67 ppm, (5H, m), 7.79 ppm (1H, d, 8.0 Hz), 8.04 ppm (2H, d, 7.5 Hz), 8.86 ppm (1H, s), 9.32 ppm (1H, d, 2.0 Hz).

### Example 1a: 3-Phenylsulfonyl-8-piperazin-1-yl-quinoline Form III

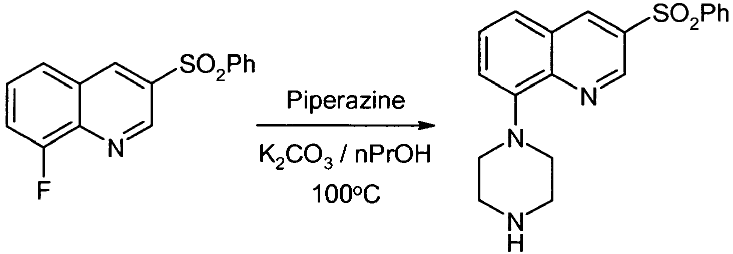

A vessel was charged with 8-fluoro-3-phenylsulfonylquinoline (20.0 g), piperazine (30.0 g), potassium carbonate (9.60 g) and n-propanol (40 ml). The mixture was stirred and heated under nitrogen at 100°C. After 23 h the reaction mixture was cooled to 95°C and seeded with Form III 3-phenylsulfonyl-8-piperazin-1-yl-quinoline (20 mg) slurried in n-propanol (2 x 0.1 ml). (See WO 05/040124 for a process for making Form III 3-phenylsulfonyl-8-piperazin-1-yl-quinoline). The reaction mixture was aged at 95°C for 15 min then cooled to 30°C over 1 hr. Water (160 ml) was added over 1 hr maintaining contents at 30-34°C. The slurry was aged at 30°C for 16 hrs then the product was collected by vacuum filtration. The bed was washed with 4:1 water / n-propanol (2 x 40 ml) and pulled dry. The product was dried *in vacuo* at 50°C to give the title compound, 21.25 g, 86% yield.
¹H NMR, CDCl₃, 400 MHz
3.17ppm (4H, t, *J* 4.5 Hz), 3.34 ppm (4H, t, *J* 4.5 Hz), 7.27 ppm (1H, dd, *J* 2.0 & 7.0 Hz), 7.49-7.60 ppm (5H, m), 8.00-8.02 ppm (2H, m), 8.76 ppm (1H, d, *J* 2.5 Hz), 9.22 ppm (1H, d, *J* 2.5 Hz).

### Example 1b: 3-Phenylsulfonyl-8-8-piperazin-1-yl-quinoline Form II

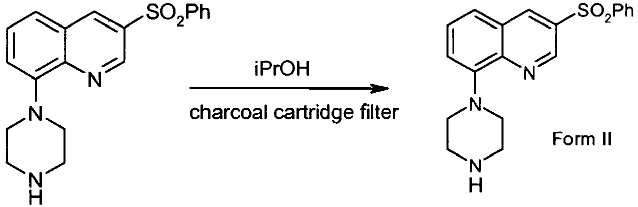

A mixture of 3-phenylsulfonyl-8-piperazin-1-yl-quinoline (813 g) and isopropanol (16.3 L) was heated at 80-82°C for 35 min then passed through a CUNO™ immobilised charcoal filter (www.cuno.com), the filter was then rinsed with refluxing isopropanol (2.4 L). The filtrate was heated to reflux to dissolve solid which has crystallised upon cooling. The resulting solution was cooled to 63°C and seeded with 3-phenylsulfonyl-8-piperazin-1-yl-quinoline, Form II (0.81 g) slurried in isopropanol (2 x 8 mL). (See WO 03/080580 for a process for making Form II 3-phenylsulfonyl-8-piperazin-1-yl-quinoline). The contents were aged at 63-61 °C for 15 min, cooled to 22°C over 3 hr 45 min then aged at 22-21 °C for a further 30 min. The contents were filtered and cake washed with isopropanol (2 x 1.2 L). The cake was pulled dry then dried at 50°C under reduced pressure to yield 3-phenylsulfonyl-8-piperazin-1-yl-quinoline, Form II, (622 g, 77%).

### Example 2: Synthesis of 3-[(3-fluorophenyl)sulfonyl]-8-(1-piperazinyl)quinoline

The following Example illustrates the preparation of 3-[(3-fluorophenyl)sulfonyl]-8-(1-piperazinyl)quinoline, but is not intended to be limiting.

Proton Magnetic Resonance (NMR) spectra were recorded on a Bruker instrument at 250 or 400 MHz. Chemical shifts are reported in ppm (δ) using tetramethylsilane as internal standard. Splitting patterns are designated as s, singlet; d, doublet; t, triplet; q, quartet; m, multiplet; br, broad. The NMR spectra were recorded at a temperature ranging from 25 to 90°C. When more than one conformer was detected the chemical shifts for the most abundant one are reported.

Chromatography was carried out on silica gel using an appropriate elution solvent system.

The following Table 1 lists some abbreviations:

| | |
|---|---|
| EtOAc | Ethyl acetate |
| DCM | dichloromethane |
| DMF | N,N-dimethylformamide |
| MeOH | Methanol |
| EDC | 1-(3-dimethylaminopropyl)-3-ethylcarbodiimide hydrochloride |
| HOBT | 1-Hydroxybenzotriazole |
| DMSO | Dimethyl sulfoxide |
| DCE | 1,2-Dichloroethane |
| MMPP | Magnesium monoperoxypthalate hexahydrate |

### Intermediate 1: 8-Chloro-3-iodoquinoline

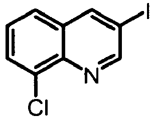

To 8-chloroquinoline (49.4 g, 301.95 mmol, Acros) in a three-necked 2L round bottomed flask was added acetic acid (300ml). N-iodosuccinimide (67.94 g, 301.95 mmol, Avocado) was added in portions to the stirred solution and the mixture was then heated at 70 °C (internal temperature) for 18h. The black mixture was allowed to cool to room temperature and then concentrated in vacuo (bath temperature 40°C). Dichloromethane (600 ml) was added and the solution was washed with aqueous 10% w/v sodium thiosulfate (2x300 ml). The organic layer was dried (MgSO₄) and the solvent was removed *in vacuo* (bath temperature 40 °C). The residue was recrystallised from ethyl acetate and gave the title compound as a yellow solid (42g, 48%).

### Intermediate 2: 8-Chloro-3-[(3-fluorophenyl)thio]quinoline

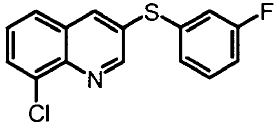

A three-necked 2L round bottomed flask (fitted with an overhead stirrer and thermometer) was charged with 8-chloro-3-iodoquinoline (intermediate 1, 42 g, 145 mmol), Cul (1.38 g, 7.25 mmol, Aldrich), K₃PO₄ (61.7 g, 290 mmol, BDH), ethylene glycol (500ml, Aldrich) and finally 3-fluorobenzenethiol (27.9 g, 218mmol, Fluorochem). The mixture was then heated at 65 °C (internal temperature) and stirred for 18h. After this time LCMS and TLC indicated approximately 20% unreacted starting material. A further portion of 3-fluorobenzenethiol (0.3 equivalents) was added and heating was continued for 4h. After allowing the mixture to cool, water (500ml) and DCM (500ml) were added. The mixture was stirred for 10min and the organic layer was separated. Charcoal was added to the organic layer and the mixture was stirred for 20min then filtered and washed with water (300ml). The organic layer was concentrated *in vacuo* to give the title compound as yellow solid (43g).

### Intermediate 3: 8-Chloro-3-[(3-fluorophenyl)sulfonyl]quinoline

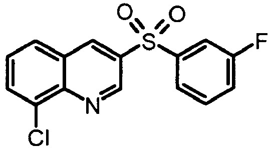

To a three-necked 2L round bottomed flask (fitted with an overhead stirrer and thermometer) was added 8-chloro-3-[(3-fluorophenyl)thio]quinoline (intermediate 2, 42 g, 145 mmol) and DCM (500ml)/MeOH (100ml). To this solution was then added in portions MMPP (161.7g, 80% tech., 326.9 mmol, Avocado) (10 portions) whilst maintaining an internal temperature below 25 °C. Once the addition was complete the resulting slurry was stirred at room temperature for 18h. A solution of 10% w/v aqueous sodium sulfite (500ml) was then added over 30min (internal temperature below 35°C) and the layers were then separated. The organic layer was washed with saturated aqueous NaHCO₃ (200ml) and then concentrated to 300ml. This mixture was stirred at 0°C (ice bath) for 30min and filtered, washing the filter cake with cold DCM (100ml). The filter cake was dried *in vacuo* at 40 °C for 4h to give the title compound as a white solid (35.7 g, 76.5%).

### Intermediate 4: 1,1-Dimethylethyl 4-{3-[(3-fluorophenyl)sulfonyl]-8-quinolinyl}-1-piperazinecarboxylate

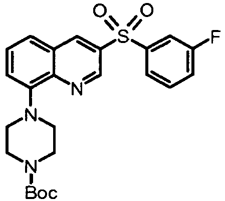

To a solution of 8-chloro-3-[(3-fluorophenyl)sulfonyl]quinoline (intermediate 3, 25 g, 77.88 mmol) in de-gassed 1,4-dioxane (1 L) in a three-necked 2 L round-bottomed flask was added 1,1-dimethylethyl 1-piperazinecarboxylate (15.96 g, 85.67 mmol), sodium t-butoxide (10.48 g, 109 mmol), *tris*-dibenzylideneacetone-dipalladium (0) (2.14 g, 2.34 mmol) and 2'-(dicyclohexylphosphanyl)-N,N-dimethyl-2-biphenylamine (2.76 g, 7.01 mmol). An overhead stirrer was fitted and the mixture heated at 60 °C (internal temperature) for 18 h. Approximately 10% conversion to desired product was observed, so further *tris*-dibenzylideneacetone-dipalladium (0) (2 mol %) and 2'-(dicyclohexylphosphanyl)-N,N-dimethyl-2-biphenylamine (6 mol %) was added and the mixture heated at 60 °C for a further 2 days. LCMS analysis indicated reaction had progressed to completion. Reaction mixture was filtered and the filtrate evaporated *in vacuo* and dried *in vacuo* for 2 days. The crude product was purified by chromatography (silica gel Flash 75 cartridge) eluting with ethyl acetate - hexane 1:4 then ethyl acetate - hexane 1:3 to give the title compound (20 g, 55%).

Residual palladium could conveniently be removed from the title compound using the following representative procedure:

The title compound (5.5 g) was dissolved in toluene (30 ml) and a solution of L-cysteine (2.8 g) in water (40 ml) added. The resulting mixture was stirred at reflux for 1.5 h then cooled to room temperature and stirred overnight. The phases were then separated and the aqueous phase extracted with toluene. The combined organic phases were then stirred again with a solution of cysteine (2.8 g) in water (40 ml) at reflux for 2 h. After cooling, the toluene phase was separated, dried over magnesium sulphate, filtered and the filtrate evaporated *in vacuo.* The product was precipitated from ether then dried in a vacuum oven at 40 °C overnight, yield 5.4 g.

### Example 2: 3-[(3-Fluorophenyl)sulfonyl]-8-(1-piperazinyl)quinoline hydrochloride

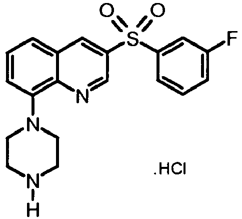

A solution of HCl in 1,4-dioxane (4 M, 330 mL) was added to 1,1-dimethylethyl 4-{3-[(3-fluorophenyl)sulfonyl]-8-quinolinyl}-1-piperazinecarboxylate (intermediate 4, 30 g, 63.7 mmol, pre-treated in a manner similar to that described above to remove palladium residues). A further aliquot of 1,4-dioxane (200 ml) was added and the resulting mixture stirred at 80 °C for 1 h. The mixture was cooled to 0 °C and the resulting yellow powder was collected by filtration and washed with cold 1,4-dioxane (2 x 150 ml) and ether (200 ml). The resulting material was recrystallised from isopropanol-water as follows. Compound was dissolved in a boiling mixture isopropanol (1050 ml) and water (125 ml) then the solution was evaporated to half its initial volume. Boiling isopropanol (500 ml) was added and the volume reduced by half again by evaporation. The resulting solution was cooled with the aid of a refrigerator. The yellow solid obtained was collected by filtration and washed with cold isopropanol (2 x 150 ml) and ether (2 x 250 ml), then dried in a vacuum oven at 40 °C to give the title compound (23.6 g, 91%).

¹H NMR (DMSO-d6): δ_{H} 3.32 (4H, m, overlapping with water signal), 3.56 (4H, m), 7.42 (1H, d, J = 8.6Hz), 7.60-7.74 (3H, m), 7.86 (1H, d, J = 7.4Hz), 7.9-8.0 (2H, m), 9.15 (1H, d, J = 2.4Hz), 9.25 (2H, br s), 9.30 (1H, d, J = 2.4Hz).

### Example 3: Effect of the 5HT6 antagonist SB742457 in the chronic inflammatory rat joint pain model.

The aim of this study was to examine whether dosing of the 5HT-₆ antagonist SB742457 was efficacious in the rat joint pain model of chronic inflammatory pain.

Random Hooded rats, 150-180g, obtained from Charles River UK, were anaesthetised under gaseous anaesthetic and the area surrounding the left and right knee joint shaved and cleaned as for aseptic surgery. Animals were injected with 150 µl of FCA into the left knee joint (intra-articular injection). Animals were immediately allowed to recover from anaesthetic in a warmed/oxygenated environment until being returned to their home cage on paper bedding. No further post-op care was provided.

Rats were tested prior to surgery and from 18 h post FCA (minimum of once weekly) for weight bearing (g) and joint diameter (mm). Weight bearing is measured by the animal's ability to place body weight across both hind paws on a Dual Channel Weight Averager which was calibrated each day prior to use using a 100g weight (Rat capacitance tester - Linton Instruments). Hind paw weight bearing data was calculated and expressed as the percentage of the contralateral paw.

The study was blinded as follows: A= 1% methylcellulose, B= 0.01 mg/kg SB742457, C= 0.1 mg/kg SB742457, D= 1 mg/kg SB427457, E= 10mg/kg SB742457, F= 30mg/kg celecoxib.

All data were expressed as mean ± s.e.m. Studies in which the positive control, celecoxib, failed to demonstrate a significant reversal of hypersensitivity were considered a failed study.

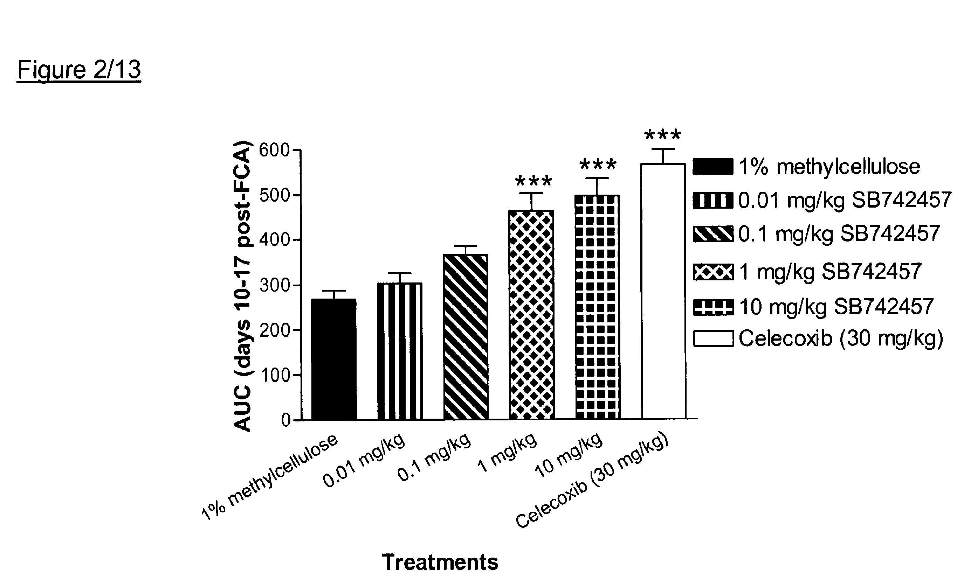

Prior to FCA intra-articular injection into the left knee, rats display even weight bearing. By day 1 post-insult weight bearing had shifted such that the majority of the body weight was transmitted through the contralateral limb. By day 10 this effect was maintained and at this point animals were randomised across treatment groups. Once daily dosing with vehicle, SB742457 or celecoxib began on day 13 and continued up to and including day 17. The 5HT6 antagonist SB742457 reversed hypersensitivity in the joint pain model of chronic inflammatory pain in rats. The effect observed showed a dose dependent reversal of hypersensitivity, with all doses above and including 0.1 mg/kg showing significant separation from vehicle on at least one day and the highest dose of 10mg/kg demonstrating effects that were not statistically different, compared with the positive control, celecoxib, as shown in Figure 1 and Table 2. The area under the curve (AUC) for control and each of the different treatment groups is shown in Figure 2.

**Table 2: Statistics for hypersensitivity data**

| | | | | | |
|---|---|---|---|---|---|
| Statistical analysis for each of the different treatment groups was carried out using ANOVA followed by Duncan's post-hoc comparisons to vehicle (p<0.05 considered significant). AUC was calculated using Microsoft Excel. | | | | | |

| Treatment/Day | 13 | 14 | 15 | 17 | AUC |
|---|---|---|---|---|---|
| 0.01 mg/kg SB742457 | | | | | |
| 0.1 mg/kg SB742457 | | | | ** | |
| 1 mg/kg SB742457 | | | *** | *** | *** |
| 10 mg/kg SB742457 | | ** | *** | *** | *** |
| 30 mg/kg Celecoxib | * | *** | *** | *** | *** |

| | | | | | |
|---|---|---|---|---|---|
| SB742457 / Celecoxib c.f. 1% methylcellulose, where *=p<0.05; **=p<0.01; ***=p<0.001 | | | | | |

These findings demonstrate an analgesic action for SB742457 in response to inflammatory pain. SB742457 has thus shown an unexpected level of efficacy in a model of chronic inflammatory pain, strongly suggesting a potential utility for this compound in the treatment of pain and in particular, chronic inflammatory pain. By virtue of the analgesic action of SB742457 in the rat model of chronic inflammatory pain, SB742457 may also be expected to be of use in the treatment of inflammatory pain associated with arthritis, for example rheumatoid arthritis or osteoarthritis.

### Example 4: Effect of the 5HT6 antagonist SB792988A in the chronic inflammatory rat joint pain model.

The aim of this study was to examine whether dosing of the 5HT-₆ antagonist SB792988A was efficacious in the rat joint pain model of chronic inflammatory pain.

Random Hooded rats, 150-180g, obtained from Charles River UK, were anaesthetised under gaseous anaesthetic and the area surrounding the left and right knee joint shaved and cleaned as for aseptic surgery. Animals were injected with 150 µl of FCA into the left knee joint (intra-articular injection). Animals were immediately allowed to recover from anaesthetic in a warmed/oxygenated environment until being returned to their home cage on paper bedding. No further post-op care was provided.

Rats were tested prior to surgery and from 18 h post FCA (minimum of once weekly) for weight bearing (g) and joint diameter (mm). Weight bearing is measured by the animal's ability to place body weight across both hind paws on a Dual Channel Weight Averager which was calibrated each day prior to use using a 100g weight (Rat capacitance tester - Linton Instruments). Hind paw weight bearing data was calculated and expressed as the percentage of the contralateral paw.

The study was blinded as follows: A= 1% methylcellulose, B= 0.1 mg/kg SB792988A, C= 1 mg/kg SB792988A, D= 3 mg/kg SB792988A, E= 10mg/kg SB792988A, F= 30mg/kg celecoxib.

All data were expressed as mean ± s.e.m. Studies in which the positive control, celecoxib, failed to demonstrate a significant reversal of hypersensitivity were considered a failed study.

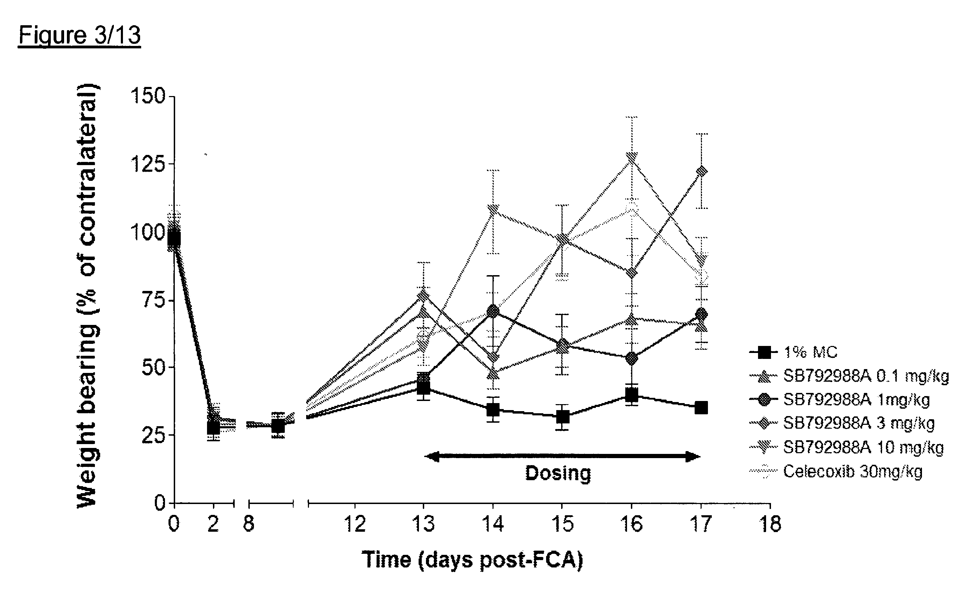

Prior to FCA intra-articular injection into the left knee, rats display even weight bearing. By day 1 post-insult weight bearing had shifted such that the majority of the body weight was transmitted through the contralateral limb. By day 10 this effect was maintained and at this point animals were randomised across treatment groups. Once daily dosing with vehicle, SB792988A or celecoxib began on day 13 and continued up to and including day 17. The 5HT-₆ antagonist SB792988A reversed hypersensitivity in the joint pain model of chronic inflammatory pain in rats. The effect observed showed a dose dependent reversal of hypersensitivity with all doses showing significant separation from vehicle on various days and the highest doses of 3 and 10mg/kg demonstrating effects that were not statistically different, compared with the positive control, celecoxib, as shown in Figure 3. The area under the curve for control and each of the different treatment groups is shown in Figure 4. AUC was calculated using Microsoft Excel.

**Table 3: Statistics for hypersensitivity data**

| | | | | | |
|---|---|---|---|---|---|
| Statistical analysis for each of the different treatment groups was carried out using ANOVA followed by Duncan's post-hoc comparisons to vehicle (p<0.05 considered significant). | | | | | |

| Treatment/Day | 13 | 14 | 15 | 16 | 17 |
|---|---|---|---|---|---|
| 0.1 mg/kg SB792988A | * | | | | * |
| 1 mg/kg SB792988A | | * | | | * |
| 3 mg/kg SB792988A | ** | | *** | * | *** |
| 10 mg/kg SB792988A | | *** | *** | *** | *** |
| 30 mg/kg Celecoxib | | * | *** | *** | *** |

| | | | | | |
|---|---|---|---|---|---|
| SB792988A / Celecoxib c.f. 1% methylcellulose, where *=p<0.05; **=p<0.01; ***=p<0.001 | | | | | |

These findings demonstrate an analgesic action for SB792988A in response to inflammatory pain. SB792988A has thus shown an unexpected level of efficacy in a model of chronic inflammatory pain, strongly suggesting a potential utility for this compound in the treatment of pain and in particular, chronic inflammatory pain. By virtue of the analgesic action of SB792988A in the rat model of chronic inflammatory pain, SB792988A may also be expected to be of use in the treatment of inflammatory pain associated with arthritis, for example rheumatoid arthritis or osteoarthritis.

### Example 5: Effect of the 5HT6 antagonist SB742457 in the FCA induced hypersensitivity rat model.

The aim of the study was to determine whether SB742457 would produce a dose-dependent reversal of FCA induced hypersensitivity.

Naïve weight bearing readings were taken. The hypersensitivity to pain was measured using the Rat incapacitance tester (Linton instruments). All rats (180-220g) received an intraplantar injection of 100ul of FCA (Freund's complete adjuvant) into the left hind paw. The FCA was sonicated for 15 minutes prior to use. 24hrs after administration of the FCA, pre-dose weight bearing readings were taken. All animals were then ranked and randomised for dosing according to their FCA window (predose difference in grams - naïve difference in grams). Rats with FCA window less than 30 were excluded from the study.

Animals were then dosed orally with either vehicle, SB42457 (0.01, 0.1, 1 and 10mg/kg p.o.) or celecoxib (10mg/kg p.o.) as appropriate according to ranking and randomisation. Animals were assessed in the weight bearing apparatus 1 hour post dose.

The study was blind and randomised by FCA window using the Latin square method. % reversals were calculated by using the naïve, pre-dose and post dose values as follows: % Reversal = [(Pre-dose - Post-dose) / (Pre-dose - Naïve)] x100.

Graphs were plotted using Prism3. Statistical analysis was carried out using ANOVA and Fischer LSD test from statistical package Statistica 6.

In this study a positive control was also tested (celecoxib). If the positive control did not produce a significant reversal of the FCA induced hypersensitivity (>60%) the experiment was deemed invalid and the study repeated.

**Table 4: Dose groups**

| Treatment | Dose mg/kg | Dose vol. ml/kg | Route | n/group |
|---|---|---|---|---|
| 100µl FCA + Vehicle | - | 5 | p.o. | n=7 (group A) |
| 100µl FCA + SB742457 | 0.01 | 5 | p.o. | n=7 (group B) |
| 100µl FCA + SB742457 | 0.1 | 5 | p.o. | n=7 (group C) |
| 100µl FCA + SB742457 | 1 | 5 | p.o. | n=7 (group D) |
| 100µl FCA + SB742457 | 10 | 5 | p.o. | n=7 (group E) |
| 100µl FCA + celecoxib | 10 | 5 | p.o. | n=7 (group F) |

**Table 5: RESULTS:**

| | |
|---|---|
| Data are expressed as percentage reversal calculated using vehicle data. Statistical analysis was carried out using ANOVA followed by a Fischer LSD test compared to vehicle. (*=p<0.05 and considered significant). | |

| 1 hour post dose | weight bearing % reversal |
|---|---|
| Vehicle p.o. | 1.58±2.01% |
| SB742457 0.01mg/kg p.o. | 27.68±6.39% |
| SB742457 0.1 mg/kg p.o. | 53.09±5.68% |
| SB742457 1mg/kg p.o. | 60.17±6.73% |
| SB742457 10mg/kg p.o. | 76.97±7.17% |
| celecoxib 10mg/kg p.o. | 74.81±8.79% |

The results show that SB742457 produced a dose-dependent reversal of FCA induced hypersensitivity in the rat, Figure 5.

### Example 6: Effect of the 5HT6 antagonists SB792988A and SB399885A in the FCA induced hypersensitivity rat model.

The aim of the study was to determine whether SB792988A would produce a dose-dependent reversal, and whether SB39985A would produce any reversal, of FCA induced hypersensitivity in the FCA induced hypersensitivity rat model. SB399885A, N-(3,5-dichloro-2-methoxy-phenyl)-4-methoxy-3-piperazin-1-yl-benzenesulfonamide hydrochloride, is known to have 5-HT₆ receptor antagonist activity (WO 02/18358, Example 2).

Naïve weight bearing readings were taken. The hypersensitivity to pain was measured using the Rat incapacitance tester (Linton instruments). All rats (200-220g) received an intraplantar injection of 100ul of FCA (Freund's complete adjuvant) into the left hind paw. The FCA was sonicated for 15 minutes prior to use. 24hrs after administration of the FCA, pre-dose weight bearing readings were taken. All animals were then ranked and randomised for dosing according to their FCA window (predose difference in grams - naïve difference in grams). Rats with FCA window less than 30 were excluded from the study.

Animals were then dosed orally with vehicle, SB-792988-A (0.01, 0.1, 1 and 10mg/kg p.o.), SB-399885-A (10mg/kg p.o.) or celecoxib (10mg/kg p.o.) as appropriate according to ranking and randomisation. Animals were assessed in the weight bearing apparatus 1 hour post dose.

The study was blind and randomised by FCA window using the Latin square method. % reversals were calculated by using the naïve, pre-dose and post dose values as follows: % Reversal = [(Pre-dose - Post-dose) / (Pre-dose - Naïve)] x100.

Graphs and were plotted using Prism3. Statistical analysis was carried out using ANOVA and Fischer LSD test from statistical package Statistica 6.

In this study a positive control was also tested (celecoxib). If the positive control did not produce a significant reversal of the FCA induced hypersensitivity (>60%) the experiment was deemed invalid and the study repeated.

**Table 6: Dose groups**

| Treatment | Dose mg/kg | Dose vol. ml/kg | Route | n/group |
|---|---|---|---|---|
| 100µl FCA + Vehicle | - | 5 | p.o. | n=7 (group A) |
| 100µl FCA + SB-792988-A | 0.01 | 5 | p.o. | n=7 (group B) |
| 100µl FCA + SB-792988-A | 0.1 | 5 | p.o. | n=7 (group C) |
| 100µl FCA + SB-792988-A | 1 | 5 | p.o. | n=7 (group D) |
| 100µl FCA + SB-792988-A | 10 | 5 | p.o. | n=7 (group E) |
| 100µl FCA + SB-399885-A | 10 | 5 | p.o. | n=7 (group F) |
| 100µl FCA + celecoxib | 10 | 5 | p.o. | n=7 (group G) |

**Table 7: Results**

| | |
|---|---|
| Data are expressed as percentage reversal calculated using vehicle data. Statistical analysis was carried out using ANOVA followed by a Fischer LSD test compared to vehicle. (*=p<0.05 and considered significant). | |

| 1 hour post dose | weight bearing % reversal |
|---|---|
| Vehicle p.o. | 2.39±6.50% |
| SB-792988-A 0.01 mg/kg p.o. | 22.91±4.13% |
| SB-792988-A 0.1mg/kg p.o. | 22.11±5.18% |
| SB-792988-A 1 mg/kg p.o. | 38.76±5.88% |
| SB-792988-A 10mg/kg p.o. | 67.00±5.08% |
| SB-399885-A 10mg/kg p.o. | 15.26±5.70% |
| celecoxib 10mg/kg p.o. | 73.00±6.01% |

The results show that SB792988A produced a dose-dependent reversal of the FCA induced hypersensitivity while SB399885A had no effect (Figure 6), thereby demonstrating that not all compounds with 5-HT₆ receptor antagonist activity are capable of reversing hypersensitivity in the FCA induced hypersensitivity rat model.

### Example 7: Effects of Alosetron, Gabapentin and Amitryptiline in the Rat Mustard Oil Model of Visceral Pain

All behavioural responses consistent with the presence of pain were determined following intra rectal injection of mustard oil in male Sprague Dawley rats. Typical behavioural responses consistent with the presence of pain following intra-colonic mustard oil injection include: arching, abdominal lifting, abdominal tensing, stretching, extending the rear leg (when lying down), raising and lowering the testicles, tip-toeing and writhing.

Male Sprague Dawley rats (130g - 160g) were briefly sedated with 50% oxygen /50% carbon dioxide and 0.2ml of 3% mustard oil injected into the colorectum, 1.5cm from the anus. The anus was plugged with vaseline and animals placed in observational cages to which they had previously been acclimatised for 45 minutes prior to mustard oil injection. The number of visceral pain related behaviours, which consisted primarily of abdominal arching, were counted over a 25 minute period and the animals culled by cervical dislocation.

The model was characterised using alosetron, gabapentin and amitriptyline, compounds known to be effective in the treatment of IBS.

In separate studies, the effects of alosetron (0.1, 0.3 and 1.0 mg/kg n=10 per group), gabapentin (10, 30 and 100 mg/kg n = 10 per group) and amitriptyline (3, 10 and 30 mg/kg n =9-10 per group) or vehicle (saline for alosetron and amitriptyline 10% 1-Methyl-2-pyrolidone in saline for gabapentin n =10 per study) given sub cutaneously 15 minutes prior to injection of 3% mustard oil were examined on pain behaviour. Results in Figures 4, 5 and 6 are expressed as mean ± sem percentage of behaviours compared to vehicle treated animals and were statistically compared to vehicle treated animals using a one way ANOVA with Dunnett's comparison, p<0.05 considered significant.

Pre-treatment with alosetron (56%, 56% and 54% reduction in behaviours compared to vehicle treated animals at 0.1, 0.3 and 1.0 mg/kg respectively), gabapentin (28%, 50% and 69% reduction in behaviours compared to vehicle treated animals at 10, 30 and 100 mg/kg respectively) or amitriptyline (43%, 73% and 91% reduction in behaviours compared to vehicle treated animals at 3, 10 and 30 mg/kg respectively) significantly reduced the number of behaviours observed following intra-rectal mustard oil, as shown in Figures 7, 8 and 9 respectively.

The results demonstrate that intra-rectal mustard oil elicits robust and reproducible pain behaviours in the conscious rat and that pre-treatment with alosetron, gabapentin or amitriptyline attenuates this behaviour, thereby demonstrating an analgesic action of these compounds which may contribute to their clinical efficacy in the treatment of visceral pain and/or IBS.

### Example 8: Effect of SB742457 in Rat Mustard Oil Model of Visceral Pain

The effect of SB742457 (0.03, 0.1, 1.0, 3.0 and 10 mg/kg, n = 10 per group for the 0.03, 0.1 and 10 mg/kg groups, and n = 20 for the 1.0 and 3.0 mg/kg groups) or vehicle (saline n = 20) given orally 60 minutes prior to injection of mustard oil (3% mustard oil, 70% ethanol in saline) were examined on pain behaviour. The total number of visceral pain related behaviours, which consisted primarily of abdominal arching, were counted over a 25 minute period and the animals culled by cervical dislocation. Behaviours were normalised as the percentage of the vehicle group mean and expressed as mean +/- sem. Data was combined from two studies and normalised as the percentage of the vehicle group mean for each individual study and expressed as mean +/- sem for both studies. Percentage of behaviours compared to vehicle treated animals were analysed statistically using a one way ANOVA with Dunnett's comparison, p<0.05 considered significant, Figure 10.

Pre-treatment with SB742457 significantly reduced the number of behaviours observed following intra-rectal mustard oil, as shown in Figure 10, (56%, 56%, 44%, 50% and 62% reduction in behaviours compared to vehicle treated animals at 0.03, 0.1, 1.0, 3.0 and 10 mg/kg respectively) and was statistically significant from vehicle treated animals at all doses examined for SB742457. The magnitude of the reduction in pain behaviour elicited by SB742457 was comparable to that produced by alosetron, gabapentin or amitriptyline.

These findings demonstrate an analgesic action for SB742457 in response to colorectal pain. SB742457 has thus shown an unexpected level of efficacy in a model of visceral pain, strongly suggesting a potential utility for this compound in the treatment of visceral pain and/or IBS.

### Example 9: Effect of SB792988A in Rat Mustard Oil Model of Visceral Pain

The effect of SB792988A (0.03, 0.1, 0.3, 1.0, 3.0 and 10 mg/kg, n = 9 for 1.0 mg/kg, n = 10 per group for the 0.03, 0.1 and 3.0 mg/kg groups, n=20 per group for the 0.3 and 10 mg/kg groups) or vehicle (saline n = 20) given orally 60 minutes prior to injection of mustard oil (3% mustard oil, 70% ethanol in saline) were examined on pain behaviour. The total number of visceral pain related behaviours, which consisted primarily of abdominal arching, were counted over a 25 minute period and the animals culled by cervical dislocation. Behaviours were normalised as the percentage of the vehicle group mean and expressed as mean +/- sem. Data was combined from two studies and normalised as the percentage of the vehicle group mean for each individual study and expressed as mean +/- sem for both studies. Percentage of behaviours compared to vehicle treated animals were analysed statistically using a one way ANOVA with Dunnett's comparison, p<0.05 considered significant, Figure 11.

Pre-treatment with SB792988A significantly reduced the number of behaviours observed following intra-rectal mustard oil, as shown in Figure 11, (38%, 40%, 42%, 55%, 65% and 74% reduction in behaviours compared to vehicle treated animals at 0.03, 0.1, 0.3, 1.0, 3.0 and 10 mg/kg respectively), and was statistically significant from vehicle treated animals at all doses examined for SB792988A. The magnitude of the reduction in pain behaviour elicited by SB792988A was comparable to that produced by alosetron, gabapentin or amitriptyline.

These findings demonstrate an analgesic action for SB792988A in response to colorectal pain. SB792988A has thus shown an unexpected level of efficacy in a model of visceral pain, strongly suggesting a potential utility for this compound in the treatment of visceral pain and/or IBS.

### Example 10: Effect of the 5HT6 antagonist SB742457 in CCl model of neuropathic pain in rats

Under Isoflurane anaesthesia, the sciatic nerve in the left leg of the rat was exposed at mid thigh level and 4 loose ligatures of Chromic 4.0 gut tied around it. The wound was closed and secured with staples. The Sham operated animals underwent the same surgical technique except that the nerve was not ligated. The animals were allowed sufficient time to recover from the surgery with special attention required to ensure that autonomy did not occur in the affected limb. The presence of mechanical (tactile) allodynia was assessed using the manual application of Von Frey hair monofilaments (Stoelting, Wood Dale, IL, USA). Animals were placed in clear Perspex boxes on a raised perforated metallic platform, from below which the monofilaments were applied in ascending order to the plantar region of the hind paw (range: 1.4g-26g). Each hair was applied for approx. 3-5 seconds until a withdrawal response was observed. The lowest hair to give a withdrawal was recorded as the response after confirmation with reapplication of lower and/or higher hairs within the range tested.

**Table 8: Dose groups**

| **GROUP** | **COMPOUND** | | **OPERATION** | **N** |
|---|---|---|---|---|
| 1 (A) | Vehicle | | CCl | 9 |
| 2 (A) | Vehicle | | Sham | 9 |
| 3 (B) | SB742457 | 10mg/kg | CCl | 9 |
| 4 (C) | Gabapentin | 30mg/kg | CCl | 9 |

The study was blinded during dosing so it was not known what the rats received until after the dosing period, when the code was broken.

All data are expressed as mean ± s.e.m. Statistical analysis was carried out using Repeated Measures ANOVA (Statistica Version 6) to compare the vehicle treated groups with the drug treated groups (P<0.05 considered significant). Area Under Curve (AUC) calculations were performed within Excel and statistical analysis was carried out using a one-way ANOVA followed by Fischer LSD post-hoc test (Statistica Version 6) to compare the vehicle treated CCI and Sham groups with the drug treated groups (P<0.05 considered significant). Data recorded in Excel (Microsoft XP) and graphs produced in GraphPad Prism (Version 4).

By 8 days post-surgery neuropathy was beginning to develop as mechanical allodynia (assessed using mVFHs) was evident in all CCI operated groups compared to the Sham operated animals. This was maintained as a stable baseline until day 23 post-surgery (Figure 12), when the animals were randomised and then chronically dosed with either SB742457 (10mg/kg b.i.d. po), gabapentin (30mg/kg b.i.d. po) or vehicle (1% methylcellulose; b.i.d. po) for 8 days (days 26-33 post-surgery). Both compounds significantly reversed CCI-induced mechanical allodynia within 1 hr of a single dose (Figure 12), which continued to develop during the dosing period with both compounds being not significantly different to Sham levels after 5 days of dosing. SB-742457 and gabapentin continued to increase, producing a maximal reversal (P<0.001) back to Sham levels after 8 days of dosing. SB742457 and gabapentin were not significantly different to each other throughout the study. Three days after cessation of treatment the withdrawal thresholds of the drug treated groups had decreased back towards vehicle treated baseline levels.

The AUC values for the drug treated groups were not significantly different to each other but were significantly different (P<0.05) to vehicle treated CCI and Sham animals (Figure 13). The AUC data reflects the time-dependent nature of the reversal of mechanical allodynia by these 2 compounds in this model.

SB742457 significantly reversed CCI-induced mechanical allodynia within 1hr of dosing, which was maintained for the duration of the dosing period. A maximal reversal back to Sham levels was achieved with this compound after 5 days of dosing, but only maintained for the remainder of the dosing period by the SB742457 treated animals. SB742457 was not significantly different to the positive control (gabapentin) group on days 1 and 5 of the dosing period and maintained this efficacy to the end of the dosing period. On cessation of treatment all groups decreased back towards vehicle treated baseline levels.

## Claims

1. Use of a compound of formula (I), or a pharmaceutically acceptable salt thereof: 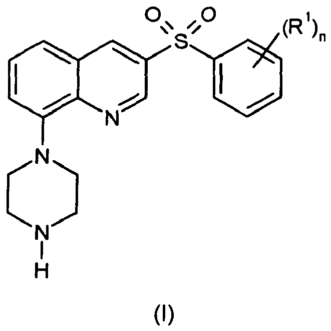 wherein:
R¹ represents a halogen atom; and
n represents 0, 1, 2 or 3;
in the manufacture of a medicament for the treatment of pain.

2. Use of a compound of formula (I) as defined in claim 1 in the manufacture of a medicament for the treatment of pain, wherein the compound of formula (I) is 3-phenylsulfonyl-8-piperazin-1-yl-quinoline or a pharmaceutically acceptable salt thereof.

3. Use of a compound of formula (I) as defined in claim 1 in the manufacture of a medicament for the treatment of pain, wherein the compound of formula (I) is 3-[(3-fluorophenyl)sulfonyl]-8-(1-piperazinyl)quinoline or a pharmaceutically acceptable salt thereof.

4. Use of a compound of formula (I) as defined in any one of claims 1 to 3, or a pharmaceutically acceptable salt thereof; in the manufacture of a medicament for the treatment of pain wherein the pain is inflammatory pain.

5. Use of a compound of formula (I) as defined in any one of claims 2 or 3 or a pharmaceutically acceptable salt thereof wherein the pain is pain associated with arthritis.

6. Use as claimed in claim 4 wherein the inflammatory pain is pain associated with rheumatoid arthritis or osteoarthritis.

7. Use of a compound of formula (I) as defined in any one of claims 1 to 3, or a pharmaceutically acceptable salt thereof, in the manufacture of a medicament for the treatment of chronic articular pain associated osteoarthritis and rheumatoid arthritis.

8. Use of a compound of formula (I) as defined in any one of claims 1 to 3, or a pharmaceutically acceptable salt thereof, in the manufacture of a medicament for the treatment of pain wherein the pain is visceral pain or neuropathic pain.

9. A compound of formula (I) as defined in any one of claims 1 to 3, or a pharmaceutically acceptable salt thereof for use in the treatment of pain in mammals.

10. A compound as claimed in claim 9 wherein the pain is inflammatory pain.

11. A compound as claimed in claim 9 wherein the pain is pain associated with arthritis and the compound is selected from 3-phenylsulfonyl-8-piperazin-1-yl-quinoline or a pharmaceutically acceptable salt thereof or 3-[(3-fluorophenyl)sulfonyl]-8-(1-piperazinyl)quinoline or a pharmaceutically acceptable salt thereof.

12. A compound as claimed in claim 10 wherein the inflammatory pain is pain associated with rheumatoid arthritis or osteoarthritis.

13. A compound of formula (I) as defined in any one of claims 1 to 3, or a pharmaceutically acceptable salt thereof for use in the treatment of chronic articular pain associated osteoarthritis and rheumatoid arthritis.

14. A compound as claimed in claim 9, wherein the pain is visceral pain or is neuropathic pain.

15. A pharmaceutical composition for use in the treatment of pain which comprises a compound of formula (I) as defined in any one of claims 1 to 3, or a pharmaceutically acceptable salt thereof, and a pharmaceutically acceptable carrier.

16. A pharmaceutical composition for use in the treatment of pain according to claim 15, wherein the pain is inflammatory pain.

17. A pharmaceutical composition according to claim 15, wherein the pain is pain associated with arthritis and the compound is selected from 3-phenylsulfonyl-8-piperazin-1-yl-quinoline or a pharmaceutically acceptable salt thereof or 3-[(3-fluorophenyl)sulfonyl]-8-(1-piperazinyl)quinoline or a pharmaceutically acceptable salt thereof.

18. A pharmaceutical composition according to claim 16, wherein the inflammatory pain is associated with rheumatoid arthritis or osteoarthritis.

19. A pharmaceutical composition as defined in any one of claims 1 to 3, or a pharmaceutically acceptable salt thereof for use in the treatment of chronic articular pain associated osteoarthritis and rheumatoid arthritis.

20. A pharmaceutical composition for use in the treatment of pain according to claim 15, wherein the pain is visceral pain or neuropathic pain.

## Patentansprüche

1. Verwendung einer Verbindung der Formel (I) oder eines pharmazeutisch verträglichen Salzes davon: 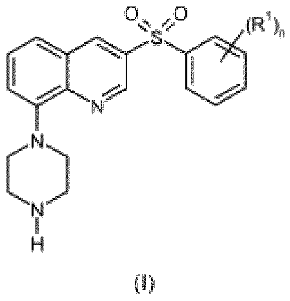 wobei:
R¹ für ein Halogenatom steht; und
n für 0, 1, 2 oder 3 steht;
bei der Herstellung eines Medikaments zur Behandlung von Schmerzen.

2. Verwendung einer Verbindung der Formel (I) wie in Anspruch 1 definiert bei der Herstellung eines Medikaments zur Behandlung von Schmerzen, wobei die Verbindung der Formel (I) 3-Phenylsulfonyl-8-piperazin-1-yl-chinolin oder ein pharmazeutisch verträgliches Salz davon ist.

3. Verwendung einer Verbindung der Formel (I) wie in Anspruch 1 definiert bei der Herstellung eines Medikaments zur Behandlung von Schmerzen, wobei die Verbindung der Formel (I) 3-[(3-Fluorphenyl)sulfonyl]-8-(1-piperazinyl)chinolin oder ein pharmazeutisch verträgliches Salz davon ist.

4. Verwendung einer Verbindung der Formel (I) wie in einem der Ansprüche 1 bis 3 definiert oder eines pharmazeutisch verträglichen Salzes davon bei der Herstellung eines Medikaments zur Behandlung von Schmerzen, wobei die Schmerzen Entzündungsschmerzen sind.

5. Verwendung einer Verbindung der Formel (I) wie in einem der Ansprüche 2 oder 3 definiert oder eines pharmazeutisch verträglichen Salzes davon, wobei die Schmerzen Schmerzen im Zusammenhang mit Arthritis sind.

6. Verwendung wie in Anspruch 4 beansprucht, wobei die Entzündungsschmerzen Schmerzen im Zusammenhang mit rheumatoider Arthritis oder Osteoarthritis sind.

7. Verwendung einer Verbindung der Formel (I) wie in einem der Ansprüche 1 bis 3 definiert oder eines pharmazeutisch verträglichen Salzes davon bei der Herstellung eines Medikaments zur Behandlung von chronischen Gelenkschmerzen im Zusammenhang mit Osteoarthritis und rheumatoider Arthritis.

8. Verwendung einer Verbindung der Formel (I) wie in einem der Ansprüche 1 bis 3 definiert oder eines pharmazeutisch verträglichen Salzes davon bei der Herstellung eines Medikaments zur Behandlung von Schmerzen, wobei die Schmerzen viszerale Schmerzen oder neuropathische Schmerzen sind.

9. Eine Verbindung der Formel (I) wie in einem der Ansprüche 1 bis 3 definiert oder ein pharmazeutisch verträgliches Salz davon zur Verwendung bei der Behandlung von Schmerzen bei Säugern.

10. Eine Verbindung wie in Anspruch 9 beansprucht, wobei die Schmerzen Entzündungsschmerzen sind.

11. Eine Verbindung wie in Anspruch 9 beansprucht, wobei die Schmerzen Schmerzen im Zusammenhang mit Arthritis sind und die Verbindung ausgewählt ist aus 3-Phenylsulfonyl-8-piperazin-1-yl-chinolin oder einem pharmazeutisch verträglichen Salz davon oder 3-[(3-Fluorphenyl)sulfonyl]-8-(1-piperazinyl)chinolin oder einem pharmazeutisch verträglichen Salz davon.

12. Eine Verbindung wie in Anspruch 10 beansprucht, wobei die Entzündungsschmerzen, Schmerzen im Zusammenhang mit rheumatoider Arthritis oder Osteoarthritis ist.

13. Eine Verbindung der Formel (I) wie in einem der Ansprüche 1 bis 3 definiert oder ein pharmazeutisch verträgliches Salz davon zur Verwendung bei der Behandlung chronischer Gelenkschmerzen im Zusammenhang mit Osteoarthritis und rheumatoider Arthritis.

14. Eine Verbindung wie in Anspruch 9 beansprucht, wobei die Schmerzen viszerale Schmerzen oder neuropathische Schmerzen sind.

15. Ein Arzneimittel zur Verwendung bei der Behandlung von Schmerzen, das eine Verbindung der Formel (I) wie in einem der Ansprüche 1 bis 3 definiert oder ein pharmazeutisch verträgliches Salz davon und einen pharmazeutisch verträglichen Träger umfasst.

16. Ein Arzneimittel zur Verwendung bei der Behandlung von Schmerzen gemäß Anspruch 15, wobei die Schmerzen Entzündungsschmerzen sind.

17. Ein Arzneimittel gemäß Anspruch 15, wobei die Schmerzen Schmerzen im Zusammenhang mit Arthritis sind und die Verbindung ausgewählt ist aus 3-Phenylsulfonyl-8-piperazin-1-yl-chinolin oder einem pharmazeutisch verträglichen Salz davon oder 3-[(3-Fluorphenyl)sulfonyl]-8-(1-piperazinyl)chinolin oder einem pharmazeutisch verträglichen Salz davon.

18. Ein Arzneimittel gemäß Anspruch 16, wobei die Entzündungsschmerzen im Zusammenhang mit rheumatoider Arthritis oder Osteoarthritis stehen.

19. Ein Arzneimittel wie in einem der Ansprüche 1 bis 3 definiert oder ein pharmazeutisch verträgliches Salz davon zur Verwendung bei der Behandlung chronischer Gelenkschmerzen im Zusammenhang mit Osteoarthritis und rheumatoider Arthritis.

20. Ein Arzneimittel zur Verwendung bei der Behandlung von Schmerzen gemäß Anspruch 15, wobei die Schmerzen viszerale Schmerzen oder neuropathische Schmerzen sind.

## Revendications

1. Utilisation d'un composé de formule (I), ou d'un de ses sels pharmaceutiquement acceptables : 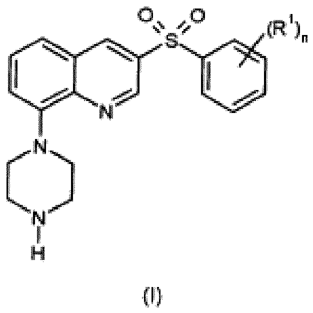 formule dans laquelle :
R¹ représente un atome d'halogène ; et
n est égal à 0, 1, 2 ou 3 ;
dans la production d'un médicament pour le traitement de la douleur.

2. Utilisation d'un composé de formule (I) tel que défini dans la revendication 1 dans la production d'un médicament pour le traitement de la douleur, dans laquelle le composé de formule (I) est la 3-phénylsulfonyl-8-pipérazine-1-yl-quinoléine ou un de ses sels pharmaceutiquement acceptables.

3. Utilisation d'un composé de formule (I) tel que défini dans la revendication 1 dans la production d'un médicament pour le traitement de la douleur, dans laquelle le composé de formule (I) est la 3-[(3-fluorophényl)-sulfonyl]-8-(1-pipérazinyl)-quinoléine ou un de ses sels pharmaceutiquement acceptables.

4. Utilisation d'un composé de formule (I) tel que défini dans l'une quelconque des revendications 1 à 3, ou d'un de ses sels pharmaceutiquement acceptables, dans la production d'un médicament pour le traitement de la douleur, la douleur étant une douleur inflammatoire

5. Utilisation d'un composé de formule (I) tel que défini dans l'une quelconque des revendications 2 et 3, ou d'un de ses sels pharmaceutiquement acceptables, dans laquelle la douleur est la douleur associée à l'arthrite.

6. Utilisation suivant la revendication 4, dans laquelle la douleur inflammatoire est la douleur associée à la polyarthrite rhumatoïde ou à l'arthrose.

7. Utilisation d'un composé de formule (I) tel que défini dans l'une quelconque des revendications 1 à 3, ou d'un de ses sels pharmaceutiquement acceptables, dans la production d'un médicament pour le traitement de la douleur articulaire chronique associée à l'arthrose et à la polyarthrite rhumatoïde.

8. Utilisation d'un composé de formule (I) tel que défini dans l'une quelconque des revendications 1 à 3, ou d'un de ses sels pharmaceutiquement acceptables, dans la production d'un médicament pour le traitement de la douleur, dans laquelle la douleur est la douleur viscérale ou la douleur neuropathique.

9. Composé de formule (I) tel que défini dans l'une quelconque des revendications 1 à 3, ou un de ses sels pharmaceutiquement acceptables, pour une utilisation dans le traitement de la douleur chez les mammifères.

10. Composé suivant la revendication 9, la douleur étant une douleur inflammatoire.

11. Composé suivant la revendication 9, la douleur étant la douleur associée à l'arthrite et le composé étant choisi entre la 3-phénylsulfonyl-8-pipérazine-1-yl-quinoléine ou un de ses sels pharmaceutiquement acceptables et la 3-[(3-fluorophényl)sulfonyl]-8-(1-pipérazinyl)quinoléine ou un de ses sels pharmaceutiquement acceptables.

12. Composé suivant la revendication 10, la douleur inflammatoire étant la douleur associée à la polyarthrite rhumatoïde ou à l'arthrose.

13. Composé de formule (I) tel que défini dans l'une quelconque des revendications 1 à 3, ou un de ses sels pharmaceutiquement acceptables, pour une utilisation dans le traitement de la douleur articulaire chronique associée à l'arthrose et à la polyarthrite rhumatoïde.

14. Composé suivant la revendication 9, la douleur étant viscérale ou étant la douleur neuropathique.

15. Composition pharmaceutique pour une utilisation dans le traitement de la douleur, qui comprend un composé de formule (I) tel que défini dans l'une quelconque des revendications 1 à 3, ou un de ses sels pharmaceutiquement acceptables, et un support pharmaceutiquement acceptable.

16. Composition pharmaceutique pour une utilisation dans le traitement de la douleur suivant la revendication 15, la douleur étant une douleur inflammatoire.

17. Composition pharmaceutique suivant la revendication 15, la douleur étant la douleur associée à l'arthrite et le composé étant choisi entre la 3-phénylsulfonyl-8-pipérazine-1-yl-quinoléine ou un de ses sels pharmaceutiquement acceptables et la 3-[(3-fluorophényl)sulfonyl]-8-(1-pipérazinyl)-quinoléine ou un de ses sels pharmaceutiquement acceptables.

18. Composition pharmaceutique suivant la revendication 16, dans laquelle la douleur inflammatoire est associée à la polyarthrite rhumatoïde ou à l'arthrose.

19. Composition pharmaceutique telle que définie dans l'une quelconque des revendications 1 à 3, ou un de ses sels pharmaceutiquement acceptables, pour une utilisation dans le traitement de la douleur articulaire chronique associée à l'arthrose et à la polyarthrite rhumatoïde.

20. Composition pharmaceutique pour une utilisation dans le traitement de la douleur suivant la revendication 15, la douleur étant la douleur viscérale ou la douleur neuropathique.
